# NEW EUROPEAN PATENT SPECIFICATION

(11) **EP 2 241 345 B2**
(45) Date of publication and mention of the opposition decision: **13.04.2022**
(45) Mention of the grant of the patent: 29.05.2013
(21) Application number: 10172075.3
(22) Date of filing: 10.04.2000
(51) Int. Cl.: A61M 16/04

(54) **Disposable Laryngeal Mask Airway Device**
Einweg-Kehlkopfmasken-Atemwegsvorrichtung
Dispositif jetable pour voie respiratoire à masque laryngé

(30) Priority: 09.04.1999 US 128469 P
(43) Date of publication of application: 20.10.2010
(62) Divisional of application: 05075463.9
(73) Proprietor: The Laryngeal Mask Company Limited, Victoria, Mahé (SC); Teleflex Life Sciences, Hamilton HM 08 (BM)
(72) Inventor: Brain, Archibald Ian Jeremy, Victoria (SC)
(74) Representative: Jacob, Reuben Ellis

(56) References cited:
- EP-A1- 0 712 638
- EP-A2- 0 732 116
- EP-A2- 0 922 465
- EP-A2- 1 579 885
- EP-A2- 2 241 345
- WO-A1-00/09189
- WO-A1-00/61213
- WO-A1-94/02191
- WO-A1-97/12640
- WO-A1-98/16273
- WO-A1-98/50096
- WO-A1-99/06093
- WO-A2-01/24860
- WO-A2-2004/089453
- DE-A1- 4 447 186
- GB-A- 2 317 830
- GB-A- 2 318 735
- GB-A- 2 321 854
- GB-A- 2 324 737
- GB-A- 2 334 215
- GB-A- 2 371 990
- US-A- 2 099 127
- US-A- 4 509 514
- US-A- 5 174 283
- US-A- 5 297 547
- US-A- 5 303 697
- US-A- 5 305 743
- US-A- 5 355 879
- US-A- 5 443 063
- US-A- 5 632 271
- US-A- 5 682 880
- US-A- 5 711 293
- US-A- 5 794 617
- US-A- 5 881 726
- US-A- 5 983 897
- US-A- 6 012 452
- US-A- 6 079 409
- US-B1- 7 156 100
- English translation of DE 4447186 A1
- Witness statement of John Paul Goff dated 18. Feb 2014
- "Clinical assessment of the single use laryngeal mask airway", the LMA Unique, British Journal of Anaesthesia, vol. 80, 1998, pages 677-679,
- "History of Anaesthesia, Oropharyngeal and Naopharyngeal airways", Can J Anaesth, vol. 43, no. 6, 1996, pages 629-635,
- The Laryngeal Mask Company Limited and LMA North America, Inc, V Ambu A/S, Ambu Inc., and Ambu Ltd., Decision of the US States Courts and Appeals for the Federal Circuit dated 21 September 2010
- Miller, Donald M: "A proposed Classification and Scoring System of Supraglottic Sealing Airways: A Brief Review", Anesth Analg, vol. 99, 2004, pages 1553-1559,
- ISO 11712 15 May 2009
- "The Glottic Aperture Seal Airway", Anesthesiology, vol. V 88, May 1998 (1998-05),
- "Impossible Insertion of the Laryngeal Mask Airway and Oropharyngeal Axes", Anesthesiology, vol. V 83, no. 4, October 1995 (1995-10),
- "A new larygneal mask prototype", Anaesthesia, vol. 50, 1995, pages 42-48,

## Description

### BACKGROUND OF THE INVENTION

The present invention relates to a laryngeal mask airway device. More specifically, the present invention relates to reduced cost laryngeal masks, improved geometric configurations for laryngeal masks, and to methods of inexpensively fabricating such masks.

The laryngeal mask airway device (LMA) is a well known device that is useful for establishing airways in unconscious patients. LMAs have been in use for about twelve years and offer an alternative to the older, even better known, endotracheal tube. For at least seventy years, endotracheal tubes comprising a long slender tube with an inflatable balloon disposed at the tube's distal end have been used for establishing airways in unconscious patients. In operation, the endotracheal tube's distal end is inserted through the mouth of the patient, past the patient's laryngeal inlet (or glottic opening), and into the patient's trachea. Once so positioned, the balloon is inflated so as to form a seal with the interior lining of the trachea. After this seal is established, positive pressure may be applied to the tube's proximal end to ventilate the patient's lungs. Also, the seal between the balloon and the inner lining of the trachea protects the lungs from aspiration (e.g., the seal prevents material regurgitated from the stomach from being aspirated into the patient's lungs).

Although they have been enormously successful, endotracheal tubes suffer from several major disadvantages. The principal disadvantage of the endotracheal tube relates to the difficulty of properly inserting the tube. Inserting an endotracheal tube into a patient is a procedure that requires a high degree of skill. Also, even for skilled practitioners, insertion of an endotracheal tube is sometimes difficult or not possible. In many instances, the difficulty of inserting endotracheal tubes has tragically led to the death of a patient because it was not possible to an airway in the patient with sufficient rapidity.

In addition to this principal disadvantage, there are also other disadvantages associated with endotracheal tubes. For example, incubation with an endotracheal tube often causes patients to suffer from severe "sore throats". The "sore throat" is principally caused by friction between the tube and the notch between the patient's arytenoid cartilages. Another disadvantage is that patients can not cough effectively while intubated with an endotracheal tube. Yet another problem with endotracheal tubes relates to the manner in which they are inserted. Inserting an endotracheal tube normally requires manipulations of the patient's head and neck and further requires the patient's jaw to be forcibly opened widely. These necessary manipulations make it difficult, or undesirable, to insert an endotracheal tube into a patient who may be suffering from a neck injury. Still another disadvantage is that endotracheal tubes provide an airway that is relatively small or narrow. The size of the airway must be relatively narrow because the distal end of the tube must be sufficiently small to fit into the trachea.

In contract to the endotracheal tube, it is relatively easy to insert an LMA into a patient and thereby establish an airway. Also, the LMA is a "forgiving" device in that even if it is inserted improperly, it still tends to establish an airway. Accordingly, the LMA is often thought of as a "life saving" device. Also, the LMA may be inserted with only relatively minor manipulations of the patient's head, neck and jaw. Further, the LMA provides for ventilation of the patient's lunge without requiring contact with the sensitive inner lining of the trachea and the size of the airway established with an LMA is typically significantly larger than the size of the airway established with an endotracheal tube. Also, the LMA does not interfere with coughing to the same extent as endotracheal tubes. Largely due to these advantages, the LMA has enjoyed increasing popularity over the last twelve years.

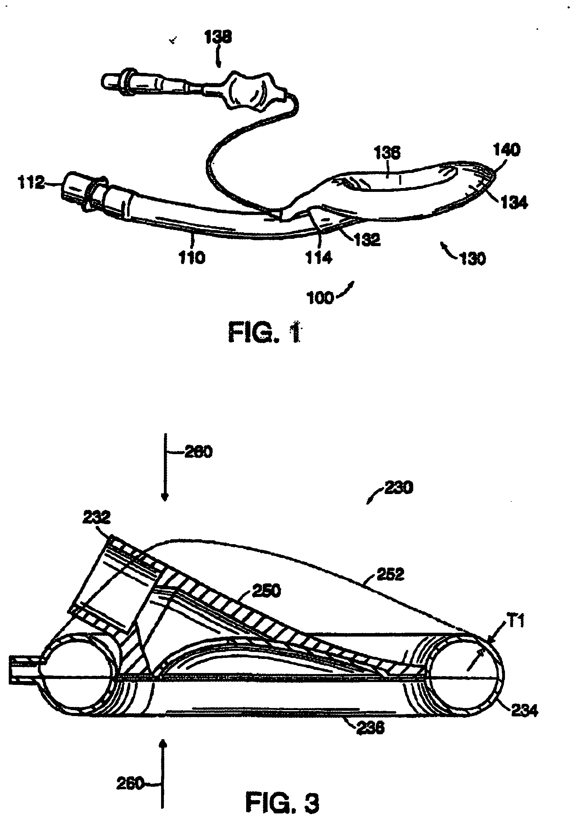

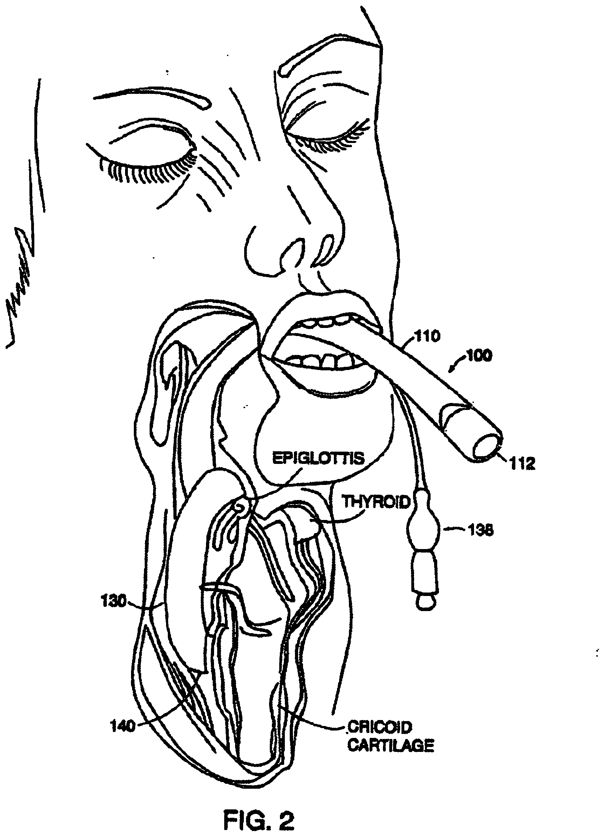

Figure 1 shows a perspective view of a prior an LMA 100 and Figure 2 illustrates an LMA 100 that has been inserted into a patient. LMAs such as LMA 100 are described for example in U.S. Patent No. 4,509,514. LMA 100 includes a flexible cylindrical tube 110 and a mask portion 130. Tube 110 extends from a proximal end 112 to a distal end 114 and mask portion 130 is coupled to the tube's distal end 114. Mask portion 130 includes a proximal end 132 and a generally elliptical inflatable cuff 134. Mask portion 130 also defines a central passageway extending from proximal end 132 to an open end 136 of cuff 134. The distal end 114 of tube 110 is telescopically fit into the proximal end 132 of mask portion 130, and LMA 100 provides a continuous, sealed, airway extending from proximal end 112 of tube 110 to the open end 136 of cuff 134. LMA 100 also includes an inflation tube 138 for selectively inflating or deflating cuff 134.

In operation, the cuff 134 is deflated, and the mask portion is inserted through the patient's mouth into the patient's pharynx. The mask portion is preferably positioned so that a distal end 140 of cuff 134 rests against the patient's normally closed esophagus and so that the open end 136 of the cuff 134 is aligned with the entryway of the patient's trachea (i.e., the patient's glottic opening). After the mask portion is so positioned, the cuff is inflated thereby forming a seal around the patient's glottic opening and this establishes a sealed airway extending from the proximal end 112 of the tube 110 to the patient's trachea.

For convenience of exposition, the term "fully inserted configuration" shall be used herein to refer to an LMA that has been inserted into a patient and has the following characteristics: (1) the mask portion is disposed around the patient's glottic opening; (2) the cuff is inflated forming a seal around the patient's glottic opening; and (3) the airway tube extends from a proximal end located outside the patient's mouth to a distal end that is coupled to the mask portion, the tube extending through the patient's mouth and the patient's natural upper airway so that the LMA provides a sealed airway extending from the tube's proximal end to the patient's lungs. Figure 2 shows an LMA in the fully inserted configuration.

When LMA 100 is in the fully inserted configuration, LMA 100 advantageously does not contact the interior lining of the trachea. Rather, the seal is established by contact between the tissues surrounding the patient's laryngeal inlet and the inflatable cuff 134. Unlike the delicate interior lining of the trachea, the tissues at the laryngeal inlet are accustomed to contact with foreign matter. For example, during the act of swallowing food, the food is normally squeezed against these tissues on its way to the esophagus. These tissues are accordingly less sensitive and less susceptible to being damaged by contact with the inflatable cuff.

Figure 3 shows a sectional side view of the mask portion 230 of another prior art LMA. The illustrated mask portion 230, which is described more fully in U.S. Patent No. 5,355,879, includes an inflatable cuff 234 and a backplate 250. Backplate 250 defines a proximal end 232 for receiving, or coupling to, a cylindrical airway tube (not shown). Mask portion 230 defines a sealed passageway, or airway, that extends from proximal end 232 through to the open end 236 of cuff 234. This mask potion 230 also includes an inflatable back cushion that, when inflated, expands to the contour illustrated by phantom outline 252. As shown in Figure 3, the cross sections of prior art cuffs are generally circular. The thickness T1 of the material used to form the cuff (i.e., the thickness of the cuff wall) is normally about 0.7 - 0.8 millimeters.

U.S. Patent No. 5,303,697 describes an example of another type of prior art LMA that is commonly known as an "intubating LMA". The intubating LMA is useful for facilitating insertion of an endotracheal tube. After an intubating LMA has been located in the fully inserted configuration, the LMA can act as a guide for a subsequently inserted endotracheal tube. Use of the LMA in this fashion facilitates what is commonly know as "blind insertion" of the endotracheal tube. Only minor movements of the patient's head, neck, and jaw are required to insert the intubating LMA, and once the intubating LMA has been located in the fully inserted configuration, the endotracheal tube may be inserted with virtually no additional movements of the patient. This stands in contrast to the relatively large motions of the patient's head, neck, and jaw that would be required if the endotracheal tube were inserted without the assistance of the intubating LMA.

U.S. Patent Nos. 5,632,271 describes an example of yet another type of prior art LMA. In addition to providing an airway tube for ventilating a patient's lungs, this LMA also provides a second tube, a drainage tube, used for draining or removing regurgitated material. The distal end of the drainage tube is disposed proximal to the normally closed entrance to the patient's esophagus. In addition to providing drainage, the drainage tube may also be used to guide insertion of a gastric tube.

EP0922465 discloses a laryngeal mask airway device in which the backplate is provided by the cuff.

In general, prior art LMAs have been manufactured by molding elastomeric materials such as silicone to desired shapes. One advantage of these materials is that they arc durable enough to permit the LMAs to be sterilized in an autoclave and reused. For example, LMAs sold by LMA International SA of Henley, England are guaranteed to survive forty sterilizations, and in practice these devices may generally be sterilized (and reused) more than forty times before becoming too worn for reuse. However, one disadvantage of these materials is that they are relatively expensive. Accordingly, it would be advantageous to develop a reduced cost LMA.

Several attempts have been made in the prior art to provide reduced cost LMAs. For example, U.S. Patent No. 6,012,452 discloses an LMA in which the mask portion is formed by adhering a foam material to both sides of the backplate. The foam forms an inflatable cuff that is attached to both sides of the plate. U.S Patent No. 5,983,897 discloses another LMA in which the mask portion is formed by attaching cuff members to the top and bottom of a backplate. The cuff members may be formed from flexible, resilient plastics material, such as PVC. One disadvantage of the LMAs disclosed in the '897 and '452 patents is that the assembly of the disclosed mask portions necessarily involves two steps: a first step of fabricating the backplate and then a second step of adhering the cuff to the top and bottom of the plate. It would therefore be advantageous to develop a process for simultaneously forming all parts of the mask portion of an LMA.

In addition to cost, another disadvantage of prior art LMAs relates to the quality of the seal established between the patient and the LMA. The LMA shown in Figure 1 generally maintains a seal up to about twenty cm H₂O. That is, when the LMA is in the fully inserted configuration, the seal between the LMA and the patient will be maintained as long as the pressure applied to the proximal end of the airway tube is less than approximately twenty cm H₂O. However, if greater pressures are applied to the proximal end of the airway tube, the seal tends to be lost thereby causing loss of some fraction of the delivered gas volume, so that positive pressure ventilation may be less effective. This stands in contrast to the endotracheal tube, which can normally maintain a seal up to fifty cm H₂O. Accordingly, it would be advantageous to provide an LMA that provides improved seals.

Still another disadvantage of prior art LMAs relates to the profile, or geometric configuration, of the deflated LMA. When the cuff of an LMA is deflated, the LMA would ideally, automatically, assume a shape that was optimized for facilitating insertion. However, prior art LMAs do not tend to automatically form such shapes when the cuff is deflated. Accordingly, several "forming tools" have been provided for affecting the shape of the deflated LMA. U.S. Patent No. 5,711,293 discloses one such forming tool. However, it would be advantageous to provide an LMA that automatically assumed a profile that facilitated insertion when the cuff was deflated.

Yet another disadvantage of prior art LMAs relates to the manner in which they are inserted into a patient. Anesthesiologists or other practitioners insert many types of prior art LMAs by pushing one of their fingers against the proximal end of the cuff. Unfortunately, this procedure requires the practitioner to insert their finger into the patient's mouth and guide the LMA past the patient's throat. Since many practitioners prefer to avoid inserting their fingers into patient's mouths, several insertion tools have been developed for facilitating insertion of various LMAs. However, it would be advantageous to provide an LMA that could be inserted without an insertion tool and without requiring insertion of a finger into the patient's mouth.

### SUMMARY OF THE INVENTION

According to the invention there is provided a laryngeal mask airway device as defined in claim 1 herein below. A method for forming the device of claim 1 is defined in claim 2.

### BRIEF DESCRIPTION OF THE FIGURES

For a fuller understanding of the nature and objects of the present invention, reference should be made to the following detailed description taken in connection with the accompanying drawings in which the same reference numerals are used to indicate the same or similar parts wherein:
Figure 1 shows a perspective view of a prior art LMA.
Figure 2 shows a prior art LMA inserted into a patient in the fully inserted configuration.
Figure 3 shows a sectional view of another prior art LMA.
Figure 4A shows a side view of an LMA constructed according to a preferred embodiment of the invention , the mask portion of the LMA being in an inflated condition.
Figures 4B and 4C show two perspective views of the LMA shown in Figure 4A.
Figure 5A shows a side view of the inflated mask portion of the LMA shown in Figures 4A, 4B, and 4C.
Figures 5B and 5C show two perspective views of the anterior portion of the mask portion shown in Figure 5A.
Figure 5D shows a perspective view of the posterior portion of the mask portion shown in Figure 5A.
Figure 5E shows a posterior view of the mask portion shown in Figure 5A.
Figure 6 shows a sectional view of the mask portion taken in the direction of line 6-6 as shown in Figure 5A.
Figure 7A shows a side view of the mask portion shown in Figures 5A when the mask portion is deflated.
Figure 7B shows an anterior view of the deflated mask portion shown in Figure 7A.
Figure 8A shows a top view of a mold that may be used to make the mask portion shown in Figure 5-7.
Figure 8B shows a sectional view of the mold taken in the direction of line 8B-8B as shown in Figure 8A.
Figures 8C and 8D show perspective views of the mold shown in Figure 8A.
Figure 9A shows a side view of the airway tube of the LMA shown in Figures 4A, 4B, and 4C.
Figure 9B shows a perspective view of the proximal section of the airway tube shown in Figures 9A.
Figures 9C and 9D show views of the proximal section taken in the direction of lines 9C-9C and 9D-9D, respectively, as shown in Figure 9B.
Figure 9E shows a side view of the integral tube and backplate section of the airway tube shown in Figure 9A.
Figures 9F and 9G show two perspective views of the integral tube and backplate section shown in Figure 9E.
Figure 10A shows a sectional view of the proximal section inserted into the integral tube and backplate section taken in the direction of the line 10A-10A as shown in Figure 9A.
Figure 10B shows a sectional view of the curved portion of the integral tube and backplate section taken in the direction of line 10B-10B as shown in Figure 9A.
Figure 10C shows a sectional view of the same component illustrated in Figure 10B when that component is subjected to external compressive forces.
Figure 10D shows a side view of an embodiment of an intubating LMA constructed according to a preferred embodiment of the invention endotracheal tube extending throush the LMA.
Figure 10E shows a sectional view of the intubating LMA taken along line 10E-10E as shown in Figure 10D.
Figure 10F shows a side view of another embodiment of an LMA constructed according to a preferred embodiment of the invention.
Figure 10G shows a perspective view of the embodiment shown in Figure 10F.
Figure 11 shows a perspective view of a tube that has formed a kink in response to bearding of the tube.
Figure 12 shows a perspective view of an LMA constructed according to a preferred embodiment of the invention in which the inflation tube has been attached to the airway tube so that the inflation tube extends into one of the grooves in the airway tube.
Figure 13 illustrates how the airway tube shown in Figure 9A deviates from its preformed configuration when the LMA is located in the fully inserted configuration.
Figure 14 shows a perspective view of the laryngeal side of the mask portion of an LMA and illustrates the regions of the mask portion that form seals with different portions of the human anatomy when the LMA is located in the fully inserted configuration.
Figure 15A shows a sectional view of a prior art LMA that has been located in the fully inserted configuration.
Figure 15B shows a sectional view of an LMA constructed according to a preferred embodiment of the invention that has been located in the fully inserted configuration.
Figure 16A shows a side view of the LMA shown in Figure 4A when the mask portion is deflated.
Figures 16B and 16C show perspective views of the LMA, with deflated mask portion, shown in Figure 16A.
Figure 17 shows an LMA constructed according to a preferred embodiment of the invention that is partially inserted into a patient.
Figure 18A shows a side view of another LMA constructed according to a preferred embodiment of the invention.
Figures 18B and 18C show perspective views of the LMA shown in Figure 18A.
Figure 18D shows a sectional view of the airway tube taken in the direction of the line 18D-18D as shown in Figure 18A.
Figure 19A illustrates how the airway tube of the LMA shown in Figures 18A-18D can be used to guide a subsequently inserted endotracheal tube.
Figure 19B shows an alternative embodiment of the LMA shown in Figures 18A-18C constructed according to a preferred embodiment of the invention in which the proximal end of the plate is not fixed to the proximal end of the backplate portion of the airway tube.
Figure 20 shows an alternative embodiment of a mask portion constructed according to a preferred embodiment of the invention.
Figure 21 is a simplified view in perspective for another LMA device constructed according to a preferred embodiment of the invention as seen in three-quarter perspective and viewing the posterior side of mask structure, in inflated condition at the distal end of an airway tube.
Figure 22 is a similar view of the structure of Figure 21, as seen from the anterior (or trachea-facing) side of the device of Figure 21, but in the evacuated state wherein thin-film marerial of the inflation is collapsed and matted against skeletal base structure of the device.
Figure 23 is a view similar to Figure 21, for an LMA device having a
   gastric-drainage feature constructed according to a preferred embodiment of the invention.
Figure 24 is a view similar to Figure 22, for the device of Figure 23.
Figure 25 is a sectional view taken generally in the longitudinal sagittal plane of the device of Figure 23, certain parts being omitted, for clarity.
Figure 26 is a plan view of the posterior side of the device of Figure 23, certain parts being omitted for clarity.
Figure 27 is a plan view as in Figure 26 but with added showing, to include structure omitted from Figure 26.
Figure 28 is a sectional view, taken at 28-28 in Figure 27.
Figure 29 is a similar sectional view, but taken at 29-29 in Figure 27.
Figure 30 is a longitudinal section as in Figure 25 constructed according to a preferred embodiment of the invention.
Figure 31 is another and similar longitudinal section, taken only to show an integrally formed feature, being a major component of the embodiment of Figure 30.
Figure 31A is a view similar to Figure 31 to show a modification.
Figure 32 is a plan view of the posterior side of the component of Figure 31.
Figure 33 is a view of a slightly modified version of the component of Figure 31.

### DETAILED DESCRIPTION OF THE PREFERRED EMBODIMENTS

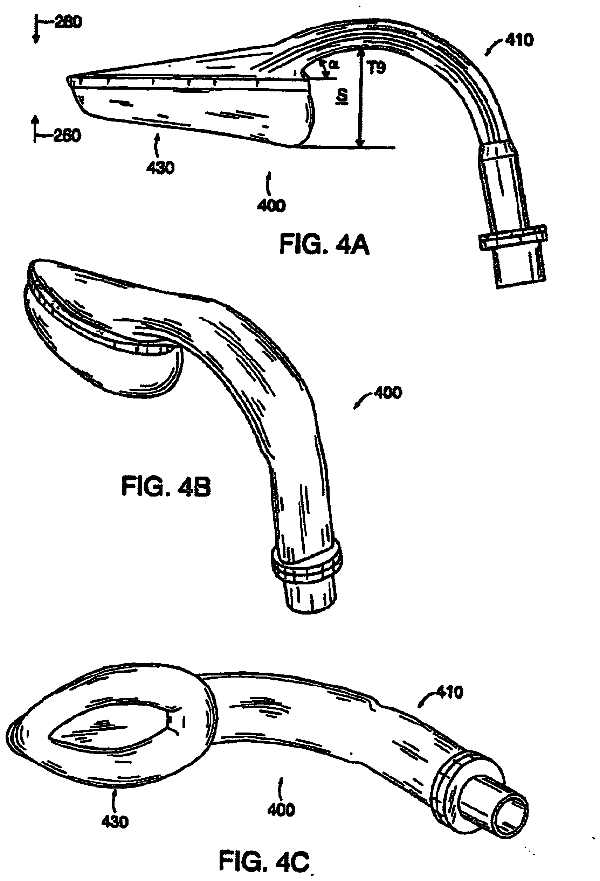

Figure 4A shows a side view of one embodiment of an LMA 400 constructed according to a preferred embodiment of the invention. Figures 4B and 4C show two perspective views of LMA 400, LMA 400 is preferably constructed from two separate pieces the are bounded, or adhered, together. The first piece is an airway tube 410 and the second piece is a mask portion 430. In Figures 4A, 4B, and 4C, the mask portion 430 is shown in an inflated condition. As will be discussed in greater detail bellow, mask portion 430 may advantageously be formed by a process called rotational molding. The airway tube 410 may also be produced by rotational molding, or alternatively, could be produced using injection or other types of molding.

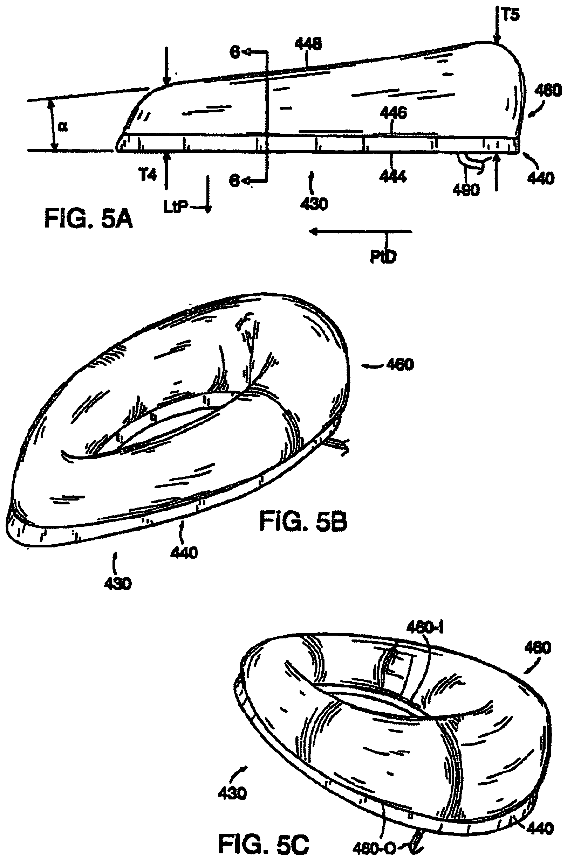

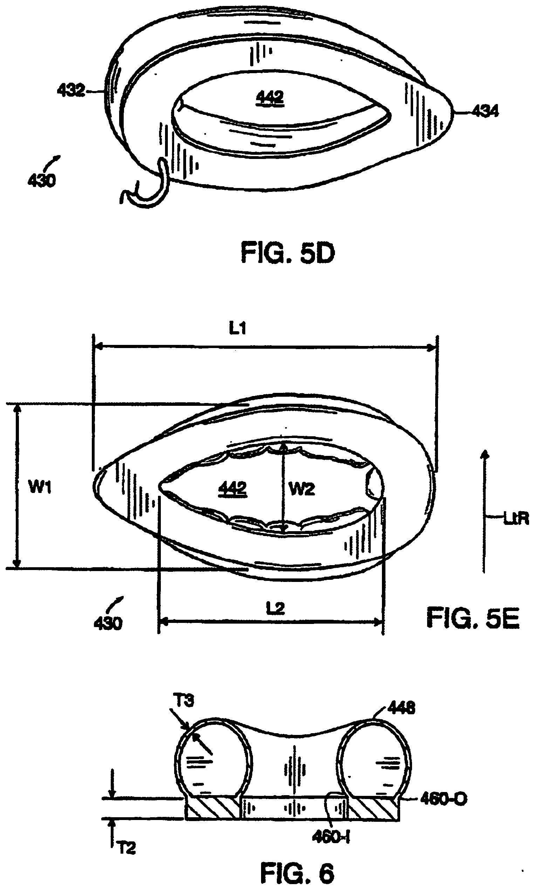

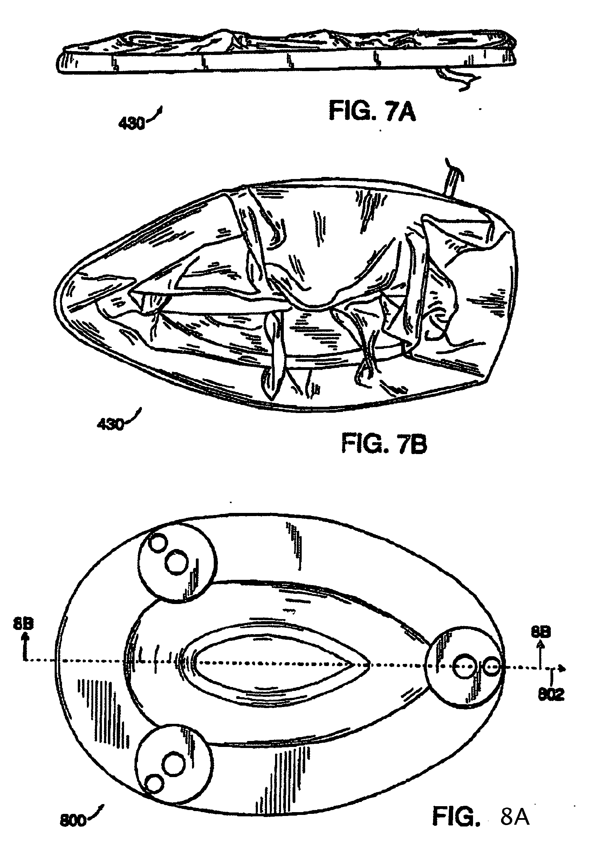

Figure 5A shows a side view of mask portion 430 when inflated. Figures 5B and 5C show two perspective views of the anterior side of mask portion 430 when inflated. Figure 5D shows a perspective view of the posterior side of mask portion 430 when inflated, and Figure 5E shows a view of the posterior side of mask portion 430 when inflated. The terms anterior and posterior as used above in connection with Figures 5B-5E are made with reference to the fully inserted configuration. That is, when the LMA 400 is in the fully inserted configuration, the portion of the mask portion 430 shown in Figures 5B and 5C will be located forward of, or anterior to, the portion shown in Figures 5D and 5E. Also, when LMA 400 is in the fully inserted configuration, the portion of mask portion 430 shown in Figures 5D and 5E will be disposed proximal to the patient's pharyngeal wall, posterior to the portions shown in Figures 5B and 5C. Figures 6 shows a sectional view of mark portion 430 taken in the direction of line 6-6 as shown in Figure 5A, Figures 7A and 7B show side and anterior views, respectively, of mask portion 430 when deflated.

Mask portion 430 includes a plate 440, an inflatable cuff 460, and an inflation tube 490. Mask portion 430 also defines a proximal end 432 and a distal end 434 (shown for example in Figure 5D). Plate 440 is characterized by a generally elliptical shape and defines a central aperture or through hole 442 (shown best in Figure 5E). For convenience of exposition, the shape of plate 440 may be referred to as that of an elliptical annulus. A classic annuls has circular symmetry, however, the elliptical annulus of plate 442 follows the elliptical profile illustrated in Figure 5E. plate 440 also defines a pharyngeal side 444 and a laryngeal side 446 (shown for example in Figure 5A). The pharyngeal side 444 of plate 440 is so named because, as will be discussed below, the pharyngeal side 444 is disposed proximal to the pharyngeal wall of a patient when LMA 400 is in the fully inserted configuration. The central aperture 442 of plate 440 extends through the entire plate from the pharyngeal side 444 to the laryngeal side 446. The distance between the pharyngeal side 444 and the laryngeal side 446 of plate 440, or the thickness of the plate, shall be referred to as T2, as shown in Figure 6. In some embodiments, the plate is substantially flat in that the thickness T2 is substantially uniform throughout the plate. One preferred value for the thickness T2 of the substantially flat plate 440 is about two millimeters plus or minus one millimeter. Even more preferably, the thickness T2 of the substantially flat plate 440 is two millimeters plus or 0.5 millimeters. Even more preferably, the thickness T2 of the substantially flat plate 440 is substantially equal to two millimeters. In other embodiments, it may be advantageous for the plate to have a tapering thickness so that the plate is thicker at the proximal end than at the distal end. For example, the thickness of the plate T2 may be about two millimeters at the proximal end and may smoothly taper to about one and a half millimeters at the distal end.

Inflatable cuff 460 is formed from a very thin, flexible, sheet of material that is attached to the laryngeal side 446 of plate 440. As shown best in Figure 6, the cross-section of cuff 460, when inflated, is generally U-shaped (or has the shape of an inverted "U"). The generally elliptical inner periphery 460-1 of cuff is sealed, or attached, to plate 440 proximal to the generally elliptical periphery of aperture 442, and the generally elliptical outer periphery 460-O of cuff 460 is scaled, or attached, to plate 440 proximal to the generally elliptical outer periphery of the plate 440. The thickness of the cuff (i.e., the cuff wall), as shown in Figure 6, shall be referred to as T3. One preferred value for the thickness T3 of the cuff is about 0.04 to 0.24 millimeters. More preferably, the thickness T3 is in the range 0.08 to 0.20 millimeters (or 0.14 plus or minus 0.06 millimeters). Even more preferably, the thickness T3 of the cuff is 0.14 plus or minus 0.03 millimeters.

For convenience of exposition, the shape of the inflated cuff 460 shall be referred to as "generally toroidarl". The shape of the cuff is not strictly a torus for several reasons. For example, the cross section of the cuff is U-shaped rather than circular (as shown in Figure 6). Also, a classic torus has a ring-like, or doughnut, shape (and is formed by rotating a circle about an axis in the plane of the circle that does not intersect the circle), whereas the cuff 460 follows the generally elliptical shape of the plate 440. Also, the thickness of the inflated cuff is not constant from the proximal end to the distal end (as shown for example in Figure 5A by the angle alpha). However, despite these variations from the classic torus, the inflated cuff may be described as having a generally toroidal configuration (since it is essentially formed by sweeping the U-shaped cross section of the inflated cuff along the elliptical contour defined by the plate 440).

Plate 440 and cuff 460 of mask portion 430 cooperate to define a generally toroidal interior volume. Inflation tube 490 extends from the pharyngeal side 444 of plate 440 through the plate and into the interior volume to permit selective inflation and deflation of cuff 460.

Like plate 440, mask portion 430 defines a pharyngeal side and a laryngeal side. The pharyngeal side of mask potion 430 is coincident with the pharyngeal side 444 of plate 440. The laryngeal side 448 of mask portion 430 is defined by inflatable cuff 460. As shown best in Figures 5A and 6, when the cuff 460 is inflated, the laryngeal side 448 of mask potion 430 is defined by the exterior surface of cuff 460 at the portion of the cuff 460 that is disposed opposite to plate 440, or furthest from plate 440. When LMA 400 is in the fully inserted configuration, the laryngeal side 448 of mask portion 430 is in physical contact with the tissues surrounding the patient's laryngeal inlet. As shown best in Figures 5D and 5E, when cuff 460 is inflated, the aperture 442 extends entirely through the mask portion so that the mask portion 430 defines a passage 442 that extends from the laryngeal side to the pharyngeal side.

For convenience of exposition, three directions shall be defined with respect to mask portion 430. The arrow PtD shown in Figure 5A extends in a proximal-to-distal direction. Mask portion 430 extends in the proximal-to-distal direction from the proximal end 432 to the distal end 434. It will be appreciated that a distal-to-proximal direction extends opposite to, or is rotated 180 degrees from, the proximal-to-distal direction. The arrow LtP shown in Figure 5A extends in a laryneal-to-pharyngeal direction. Mask portion 430 extends in the laryngeal-to-pharyngeal direction from laryngeal side 448 to pharyngeal side 444. It will be appreciated that a pharyngeal-to-laryngeal direction extends opposite to, or is roated 180 degrees from, the laryngeal-to-pharyngeal direction. (The laryngeal-to-pbaryngeal direction could also be referred to as the "antero-posterior" direction.) The arrow LtR shown in Figure 5E extends in the left-to-right direction. It will be appreciated that a right-to-left direction extends opposite to, or is rotated 180 degrees from, the left-to-right direction. These directions are so named because when the LMA 400 is inserted into a patient, the LMA will extend from a left side to a right side within the patient. These right-to-left and left-to-right directions could also be referred to as "lateral" directions. The proximal-to-distal, laryngeal-to-pharyngeal, and and left-to-right directions are mutually orthogonal and provide a convenient reference coordinate system for describing the LMA.

As shown in Figure 5A, the thickness of the inflated mask portion at the distal end 434 (i.e., the distance between the pharyngeal side 444 and the laryngeal side 448 of mask portion 430 as measured in the laryngeal-to-pharyngeal direction) shall be referred to as T4, and the thickness of the inflated mask portion at the proximal end 432, as measured in the laryngeal-to-phayngeal direction, shall be referred to as T5. Preferred values for T4 and T5 in female adult sizes are about 12.7 and 25.5 millimeters, respectively. (It will be appreciated that external dimensions such as T4 and T5 would be about thirteen percent larger in an adult male size of the LMA. Unless otherwise stated, dimensions discussed herein will be for the female adult size.) The profile of cuff 460 is preferably smoothly tapered as shown in Figure 5A so that the thickness of the mask portion 430 smoothly decreases from the proximal end 432 to the distal end 434. This tapering can be described in terms of the angle alpha between the pharyngeal side 444 and the laryngeal side 448 of mask portion 430, as shown in Figure 5A. One preferred value for the angle alpha is about ten degrees plus or minus one degree. More preferably, the angle alpha is ten degrees plus or minus half a degree. Most preferably, the angle alpha is substantially equal to ten degrees. As will be discussed below, this angle alpha is selected to match the human anatomy to allow all portions of the inflated cuff to contact the tissues surrounding the laryngeal inlet and to thereby provide improved seals.

The plate 440 shown in Figure 5A is characterized by a substantially constant thickness. That is, the thickness T2 (as shown in Figure 6) of plate 440 is substantially constant from the proximal end of the mask portion to the distal end of the mask portion and the variation in the mask portion's thickness is entirely provided by the cuff 460. However, as mentioned above, in some embodiments, it may be advantageous to provide plate 440 with a tapering thickness so that the distal end of the plate is thinner than the proximal end.

As shown in Figure 5E, the length of the plate 440, or the distance between the proximal end 432 and the distal end 434 as measured in the proximal-to-distal direction, shall be referred to as L1, and the length of aperture 442 as measured in the proximal-to-distal direction shall be referred to as L2. The width of the plate 440, as measured in the left-to-right direction, shall be referred to as W1, and the width of the aperture 442 as measured in the left-to-right direction shall be referred to as W2. In adult sizes of LMA 400, preferred values for L1, L2, W1, and W2, are 90, 59, 47, and 26 millimeters, respectively.

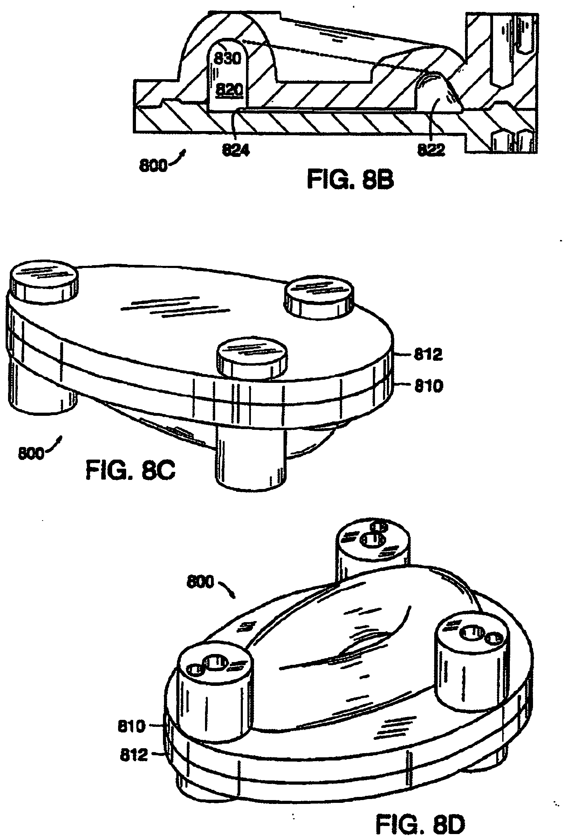

As stated above, mask portion 430 may be formed by a process called rotational molding. Figure 8A shows a top view of a mold 800 that may be used to produce mask portion 430 by rotational molding. Figure 8B shows a sectional view of mold 800 taken along the line 88-88 as indicated in Figure 8A. Figures 8C and 8D show perspective views of mold 800. As shown in Figure 8A, the mold 800 is symmetric about an axis 802. As shown best in Figures 8C and 8D, mold 800 includes a top piece 810 and a bottom piece 812. When the top piece 810 and bottom piece 812 are bolted or clamped together, they cooperatively define a hollow interior volume 820 as shown in Figure 8B. Interior wells 830 of the mold 800 define the boundaries of hollow interior volume 820.

One portion 822 of the interior volume 820 has a generally toroidal shape corresponding to the generally toroidal shape of the inflated cuff 460. Another potion 824 of the interior volume 820 has a generally elliptical shape corresponding to the shape of plate 440. That is, portion 824 defines a hollow volume, the shape of which is substantially identical to the flat, elliptical shape of plate 440. Similarly, the potion 822 defines a hollow volume, the shape of which is substantially identical to the shape of the inflated cuff 460.

In operation, mask portion 430 may be formed by adding or injecting a liquid plastic material (e.g., polyvinyl chloride or "PVC") into the interior volume 820 of mold 800 and by then rotating or otherwise moving mold 800 so as to coat the interior walls 830 with the liquid plastic material. Preferably, the mold 800 is simultaneously rotated about two axes met are at ninety degrees to each other (e.g., axis 802 and another axis that is perpendicular to axis 802). While the mold 800 is rotating, centrifugal forces cause the liquid plastic material to coat all portions of the interior walls 830 of mold 800. After all portions of the interior walls 830 have been so coated, the mold is then preferably held stationary in the position illustrated in Figure 8B. That is, the mold 800 is preferably oriented so that the portion 824 of the hollow interior 820 is at the bottom of the mold (i.e., so that portion 824 is parallel to the ground and is closer to the ground, or lower, than any other portion of the hollow interior 820) while the mold 800 is held stationary. While the mold 800 is held in this stationary position, most of the liquid plastic material drains, or flows, down along the interior wells 830 into the portion 824. However, all of the liquid plastic material does not flow into portion 824. Rather, surface tension or other forces cause a thin coating of the liquid plastic material to remain in contact with the interior walls 830 that define the portion 822. The mold 800 is preferably held stationary long enough for the plastic material to cure and solidify before the mold is opened by separating the top and bottom pieces 810, 812.

The material that filled portion 824 forms the plate 440 of the mask portion 430. The thin coating of plastic material that lined the interior walls 830 of portion 822 forms a cuff 460 that is integrally attached to the plate 440. Air trapped within the interior volume 820 while the mask portion 430 is being formed becomes trapped within the cuff 460. So, when the mask portion 430 is removed from mold 800, the cuff 460 is partially inflated. The cuff 460 is only partially inflated (rather than fully inflated) when the mask portion 430 is removed from mold 800 because, as the mold cools, the trapped air shrinks in volume and accordingly only partially fills the interior volume defined by the cuff 460.

It will be appreciated that a variety of materials may be introduced into the mold 800 and used to form mask portion 430. The term liquid plastic material is used herein to refers to any material that is capable of curing from a liquid or fluid state to a solid, flexible or plastic, state. Due to its flexibility, resistance to stretching, and ability to define complex shapes such as that of inflated cuff 460, polyvinyl chloride is a preferred material to use as the liquid plastic material that forms mask portion 430. However, it will be appreciated that other materials could also be used.

Once the mold 800 has been opened and the cured plastic plate and cuff have been removed, fabrication of mask portion 430 may be completed by adding inflation tube 490. It will be appreciated that adding inflation tube 490 is a relatively simple step and is accomplished by forming an aperture in plate 440 that extends from the pharyngeal side 444 through the plate and into the interior volume defined by cuff 460, and then fixing inflation tube 490 to that aperture. Alternatively, as will be discussed below, it may sometimes be advantageous to provide a mask portion 430 that does not include an inflation tube. In these cases, fabrication of the mask portion is complete as soon as the cured, integrally formed, plate 440 and cuff 460 have been removed from the mold 800.

The cured mask portion is preferably relatively soft and flexible. The durometer of the cured mask portion 430 is fifty four plus or minus ten on the shore A scale of hardness.

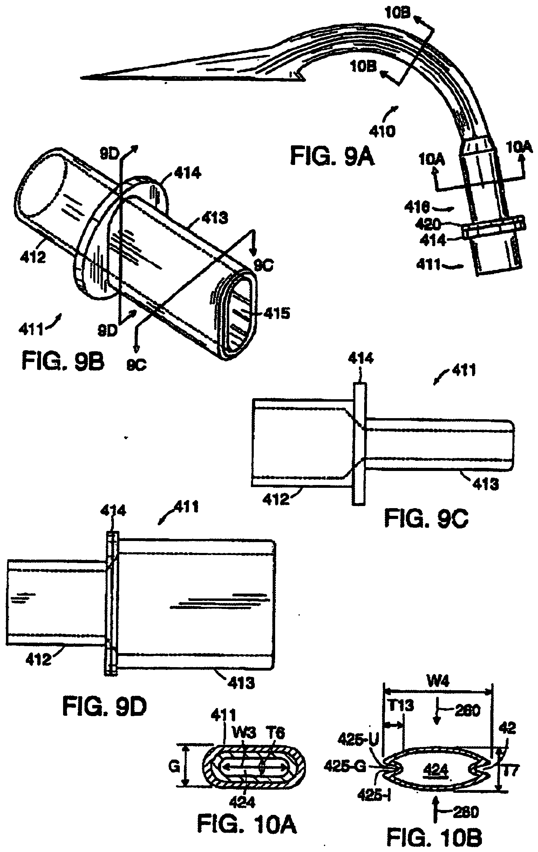

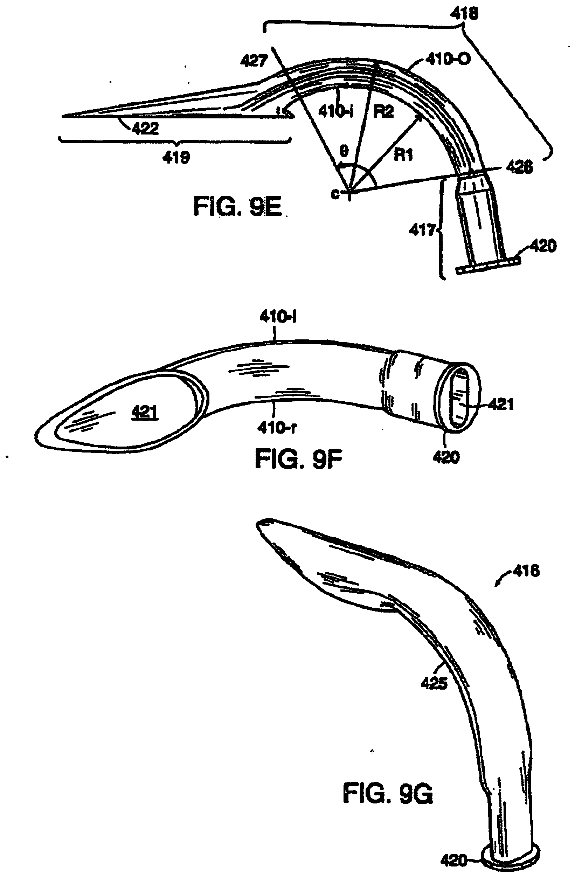

Figure 9A shows a side view of airway tube 410, which includes a connector section 411 and an integral tube and backplate section 416. Figure 9B shows a perspective view of connector section 411. Figures 9C and 9D show views of connector section 411 taken in the directions indicated by lines 9C-9C and 9D-9D, respectively, as shown in Figure 9B. Figure 9E shows a side view of integral tube and backplate section 416. Figures 9F and 9G show two perspective views of integral tube and backplate section 416.

Referring to Figures 9B, 9C, and 9D, connector section 411 includes a proximal portion 412 and a distal portion 413. Proximal portion 412 is preferably cylindrical and configured to couple to standard medical ventilating, or anaesthetic devices. Distal portion 413 is preferably oblong as shown best in the perspective view of Figure 9B. Connector section 411 further includes a disk shaped plate, or flange, 414 that extends around the junction of proximal potion 412 and distal portion 413. Connector section 411 also defines a sealed internal airway passage 415 that extends entirely through the proximal portion 412 and the distal portion 413. In the proximal portion 412, the cross section of the passage 415 is circular, and in the distal portion 413, the cross section of the passage 415 is oblong.

Referring to Figures 9E, 9F, and 9G, integral airway tube and backplate section 416 includes a proximal portion 417, a central or curved portion 418, and a backplate portion 419. A disk shaped plate, or flange, 420 is integrally attached to the proximal end of proximal portion 417. Section 416 defines a hollow internal passage 421 that extends entirely through the proximal, curved, and backplate portions 417, 418, 419.

Airway tube 410 is assembled by coupling the connector section 411 and the integral airway tube and backplate section 416 together. As shown in Figure 9A, when the parts are so coupled, the flange 414 of connector section 411 abuts the flange 420 of section 416. Also, the distal portion 413 of connector section 411 extends telescopically into the portion of internal passage 421 that is defined by proximal portion 417 of section 416. Also, the internal passage 415 of connector section 411 communicates with the internal passage 421 of section 416 so that the airway tube 410 defines a continuous sealed internal passage 424 (shown for example in Figures 10A and 10B) that extends from the tube's proximal end to the tube's distal end. Airway tube 410 also defines a left side 410-1, a right side 410-r (shown for example in Figure 9F), an inner side 410-i, and an outer side 410-o (shown for example in Figure 9E). Note that the left and right sides are defined with respect to a person (e.g., a physician) that is inserting the LMA into a patient and that the left side 410-1 of the tube will actually be disposed on the right side of the patient's natural airway when the LMA is in the fully inserted configuration.

Backplate portion 419 defines a laryngeal side 422 and a pharyngeal side 423. When the LMA 400 is assembled, the laryngeal side 422 of bookplate portion 419 is attached or fixed to the pharyngeal side 444 of mask portion 430. Also, when the assembled LMA 400 is in the fully inserted configuration, the pharyngeal side 423 of the backplate portion 419 contacts the pharyngeal wall of the patient When LMA 400 is assembled, the internal passage 424 of tube 410 communicates with the passage defined by mask portion 430 and the LMA 400 defines a sealed airway passage that extends from the proximal end of the tube 410 to the central aperture 442 of mask portion 430.

The airway tube 410 is sized so that when the LMA is in the fully inserted configuration, the proximal portion 417 of the airway tube will be disposed between the patient's upper and lower teeth. Figure 10A shows a cross-sectional view of the proximal section 417 into which the connector section 411 has been inserted taken along the line 10A-10A as shown in Figure 9A. The airway tube 410 is also sized so that when the LMA in the fully inserted configuration, the central portion 418 will extend through the patient's natural upper airway between the laryngeal inlet and the patient's teeth. Figure 10B shows a cross sectional view of the central portion 418 taken along the line 10B-10B as shown in Figure 9A. As shown in Figure 10B (as well as Figures 9A and 9E), airway tube 410 defines longitudinal folds 425 that extend along the left and right sides of the central and backplate portions 418,419.

Connector section 411 and integral tube and backplate section 416 of airway tube 410 are preferably formed using molding techniques such as injection or rotational molding. In one preferred embodiment, connector section 411 is formed from polycarbonate and the material of section 411 is characterized by a durometer of 95 Shore A. Integral tube and backplate section 416 is preferably formed from a flexible plastic material (e.g. PVC) and has a durometer of 70 plus or minus 15 Shore A.

Connector section 411 is preferably relatively hard so that (1) it is easy to reliably attach the proximal portion 412 of section 411 to standard breathing apparatus and (2) patient's can bite down on the distal portion 413 without causing collapse or shrinkage of the internal airway passage provided by section 411. Note that when the LMA is in the fully inserted configuration, the patient's teeth will contact proximal portion 417 of the integral tube and backplate section rather than section 411, because the distal portion of section 411 extends into the proximal portion 417 as illustrated in Figure 9A. However, pressure applied by the patient's teeth will be transferred to section 411, and section 411 is preferably sufficiently hard to resist this pressure without allowing the internal peerage 415 to collapse.

Section 416 is preferably softer than section 411 to facilitate bending the section 416 as necessary to insert the LMA into a patient and to permit unhindered flexion and extension of the patient's neck while LMA 400 is in the fully inserted configuration. However, as will be discussed below, section 416 is preferably stiff enough, at least at room temperature, so that LMAs constructed according to the invention may be inserted by applying pressure to section 416 without requiring insertion of a finger into the patient's mouth.

Retuning to Figures 4A-4C, it can be seen that LMA 400 may be formed by fixing or attaching the airway tube 410 to the mask portion 430. More specifically, the laryngeal side of the backplate portion of the airway tube is attached to the pharyngeal side of the mask portion so that the outer perimeter of the laryngeal side 422 of the backplate portion surrounds the central aperture 442 of the plate 440. The airway tube 410 may be attached to the mask portion 430 by heat sealing, gluing, or otherwise bonding or fixing the two components together.

As shown for example in Figure 9F, the backplate portion 419 defines a "dome shard" or "bowl shaped" interior volume. When the backplate portion 419 is attached to the mask portion 430, the backplate portion 419 and mask portion 43 cooperatively define a hollow bowl shaped interior volume as shown for example in Figure 4C. As will be discussed below, portions of the larynx extend into this bowl shaped volume when the LMA is in the fully inserted configuration,

One advantage of LMA 400 is that it is relatively simple and inexpensive to produce. As discussed above, both the mask portion 430 and the airway tube 410 may be produced using a rotational molding process. The airway tube 410 may alternatively be produced using injection molding. Each of these steps (i.e., producing the mask portion 430 and producing the airway tube 410) is relatively simple and inexpensive. Fabrication of the LMA 400 may be completed by adding an inflation tube to mask portion 430 (in embodiments that use inflation tubes) and by attaching the airway tube 410 to the mask portion 430. Accordingly, LMAs 400 may be fabricated at very low cost. Ths low cost of fabrication enables LMAs constructed according to the invention be used as disposable devices. That is, the economics of constructing LMAs according to the invention, such as LMA 400, enable them to be used once and then discarded.

Several structural advantages of LMAs constructed according to the invention will now be discussed. As shown for example in Figures 4A-4C and 9A, the backplate portion 419 essentially forms a backplate of the LMA 400. In most prior art LMA constructions (e.g., as shown in Figure 3), the mask portion includes a backplate and defines a cylindrical aperture for receiving, or or connecting with, a cylindrical airway tube. Forming the mask portion an added backplate disadvantageously increases (1) the mechanical complexity of the mask portion and (2) the cost of the mask portion. Also, the junction, which is found in prior an LMAS, of a cylindrical airway tube and a cylindrical aperture in a backplate tends to form a relatively stiff construction. For example, in the LMA illustrated in Figure 3, it is relatively difficult to compress the junction of the cylindrical airway tube and the bookplate in the direction indicated by arrows 260. Accordingly, this pardon of prior art LMA constructions disadvantageously forms a relatively thick, incompressible, structure that must be pushed between the patient' upper and lower teeth and past the patient's throat to insert the LMA. In contrast to those prior art constructions, the mask portions of LMAs constructed according to the invention are formed without backplates (e.g., as shown in mask portion 430 in Figures 5A-5D) and the backplate of the LMA is provided by the airway tube. It is less complex, and less expensive, to provide the backplate as part of the airway tube. Also, eliminating the telescopic junction of two cylindrical components that characterized the prior art make LMAs constructed according to the invention more compressible and easier to insert into into patients. For example, referring to Figure 4A, the backplate of LMA 400 compresses in the direction indicated by arrows 260 more easily than prior an LMAS. This facilitates pushing LMAs constructed according to the invention between the patient's upper and lower teeth and past the patient's throat.

In addition to providing a backplate, the general shape of the airway tube 410 distinguishes LMA 400 from prior art LMAs. In most prior art LMAs (e.g., as shown in Figures 1 and 3), the airway tube is cylindrical. While cylindrical airway tubes have functioned well for many years in many different models of LMAs, the cylindrical configuration has some disadvantages. One critical feature for an airway tube of any LMA is the size of the internal airway passage. This passage must be large enough to provide adequate ventilation of the patient's lungs. That is, moderate pressure differentials (e.g., a pressure drop of one to two cm H₂O) between the proximal and distal ends of the airway tube be sufficient for moving a volume of air through the tube that is sufficiently large for adequately ventilating the patient's lungs. With a cylindrical airway tube it is easy to calculate the volume of air that can be moved through the tube for any given pressure differential, and the volume can be adjusted simply by adjusting (i.e., increasing or decreasing) the radius of the internal airway passage.

However, one constraint that should be considered in the design of airway tubes is that these tubes will extend through the patient's mouth, between the patient's upper and lower teeth, for as long as the LMA remains in the fully inserted configuration. So, while an LMA is inserted into a patient, the patient's mouth must remain opened wide enough to create an inter-dental gap (i.e., space between the upper and lower teeth) that is big enough to accommodate the airway tube. Holding the mouth open for long periods of time so as to create a large inter-dental gap can cause discomfort to the patient post operatively. More importantly, some patients cannot open their mouths wide enough to permit easy insertion of adequate sized cylindrical tubes. Accordingly, one disadvantage of cylindrical airway tubes is that they require a larger inter-dental gap than would a tube that had a flatter, or more oblong, cross section.

Another constraint that should be considered in the design of airway tubes is that these tubes will extend through the patient's natural upper airway for as long as the LMA remains in the fully inserted configuration. This natural, or anatomical, upper airway, which is formed by several anatomical structures including the pharyngeal wall, hard and soft palates, and tongue, is not itself cylindrical. Accordingly, a cylindrical airway tube does not form a "good fit" with the anatomical upper airway. For example, when a cylindrical tube is extended through the anatomical upper airway, the tube tends to only contact isolated portions of the anatomical structures that define the anatomical upper airway. Accordingly, more pressure is applied to those stucturres, and those structures are subjected to more trauma, than would be the case if the shape of the tube better matched the shape of the anatomical upper airway.

As shown in Figures 9A, 9E, 9F, and 9G, the proximal and central portions 417, 418 of the airway tube 410 are oblong or flattened rather than cylindrical. As will be discussed in greater detail below, this advantageously (1) maximizes the size of the tube's internal airway passage; (2) minimizes the intra-dental gap required for accommodating the airway tube; and (3) allows the tube to fit well within, or match, the patient's natural airway.

As stated above, the airway tube 410 is sized so that the proximal section 417 will be disposed between the patient's upper and lower teeth when the LMA is in the fully inserted configuration, As shown in Figure 10A, the inter-dental gap G required to accommodate proximal section 417 is narrower than would be required if the proximal section 417 were cylindrical. Rather than a circular cross section, the cross section of the internal airway passage 424 is oblong. In one preferred embodiment, the thickness G of the proximal section 417 is is about 13.0 millimeters. The cross-sectional area of the internal passage defined by airway tube 410 is preferably at least as large as that of a cylindrical tube with a nine millimeter internal diameter passage. As shown in Figure 10A, the width of the internal passage 424 may be referred to as W3 and the thickness of the internal passage 424 may be referred to as T6. In one preferred embodiment W3 and T6 are 20.0 and 6.7 millimeters, and 6.7 millimeters, respectively.

As also stated above, the airway tube 410 is sized so that the central portion 418 will extend through the patient's anatomical upper airway while the LMA is in the fully inserted configuration. As shown in Figure 10B, the cross-section of the central portion 418 is oblong rather than cylindrical. Accordingly, the central portion 418 provides a "better fit" to the anatomical airway than do cylindrical tubes. As shown in Figure 10B, the width of the central portion of the airway tube may be referred to as W4 and the thickness of the central portion of the airway tube may be referred to as T7. One preferred value for W4 is 23.7 millimeters plus or minus 10 percent (or plus or minus 2.37 millimeters) and one preferred value for T7 is 10.3 millimeters plus or minus 10 percent (or plus or minus 1.03 millimeters). More preferably, W4 and T7 are equal to 23.7 millimeters plus or minus 5 percent and 10.3 millimeters plus or minus 5 percent, respectively. Even more preferably, W4 and T7 are substantially equal to 23.7 millimeters and 10.3 millimeters, respectively. Also, the width W4 of the central portion of the airway tube is preferably equal to the thickness T7 times a factor of two, plus or minus ten percent (i.e., *W*4=(2±0.2)·*T*7). More preferably, the width W4 is equal to the thickness T7 times a factor of two, plus or minus five percent (i.e., *W*4=(2±0.1)·*T*7).

As shown in Figure 2, the airway tube of any LMA must follow a curve (about an axis extending in the left-to-right direction) from the point where it couples to the mask portion to the point where the patient's teeth contact the tube. This curve enables the tube to extend through the patient's natural upper airway from the teeth to the laryngeal inlet. One important design consideration for an airway tube of any LMA is that the airway tube should be designed so that it does not form "kinks" when it is bent, or curved, as necessary for inserting the LMA into a patient.

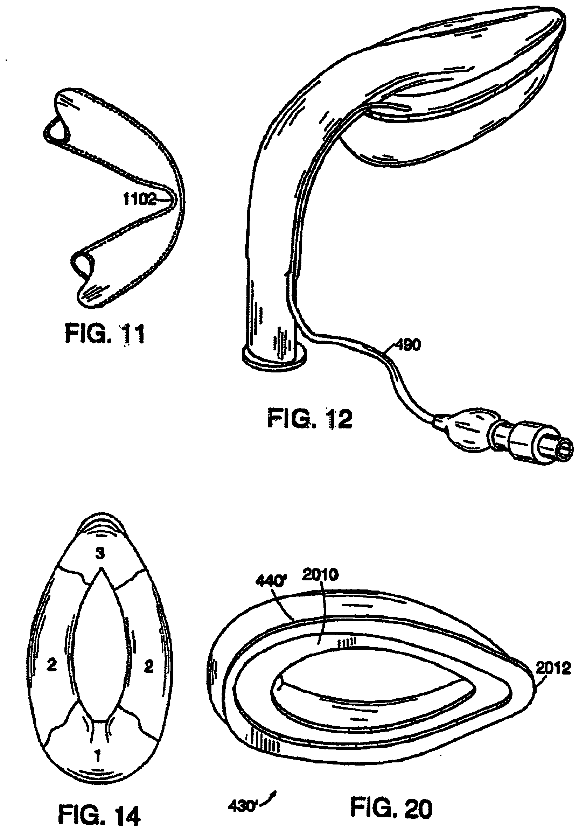

Figure 11 shows an example of a tube that has formed a kink 1102 as a result of bending the tube by an extreme amount. As is well known, the size of the internal passageway defined by any tube is dramatically decreased at any such kinks 1102. The effects of kinks in tubes is commonly experienced in connection with garden hoses. For example, formation of a single kink in a garden hose can dramatically decrease the amount of water that can pass through the hose and be distributed by a sprinkler. The effects of kinks are similar in LMAs. Any kinks forming in the airway tube of an LMA essentially close off the tube's airway passage and dramatically decrease the volume of air that can pass through the tube. Accordingly, it is very important to design the airway tube so that kinks in the tube do not form when the tube is inserted into a patient.

One advantage of cylindrical airway tubes over tubes with flatter, or more oblong, cross sections is that for any given amount of bend, the cylindrical tube is less likely to form a kink. To reduce the risk that airway tube 410 forms any kinks, tube 410 is preferably provided with two longitudinal folds 425 that extend along the left and right and sides of the tube's central and backplate portions 418, 419. As shown in Figure 10B, the cross-section of the longitudinal fold 425 that extends along the left side of the airway tube defines a recess, or groove 425-g that extends from the left exterior edge of the airway tube towards the center of the tube in the left-to-right direction. Similarly, the cross-section of the fold 425 that extends along the right side of the airway tube defines a recess that extends from the right exterior edge of the airway tube towards the center of the tube in the right-to-left direction. Each of the recesses defines an upper exterior surface 425-u and a lower exterior surface 425-1. The thickness of the longitudinal folds 425 (i.e., the thickness as measured in a direction extending from the inner side 410-i to the outer side 410-o of the airway tube) may be referred to as T 12 and the thickness of the longitudinal folds 425 as measured in the left-to-right direction may be referred to as T13. In one preferred embodiment, the thickness T12 and T13 are about three millimeters and 2.7 millimeters, respectively.

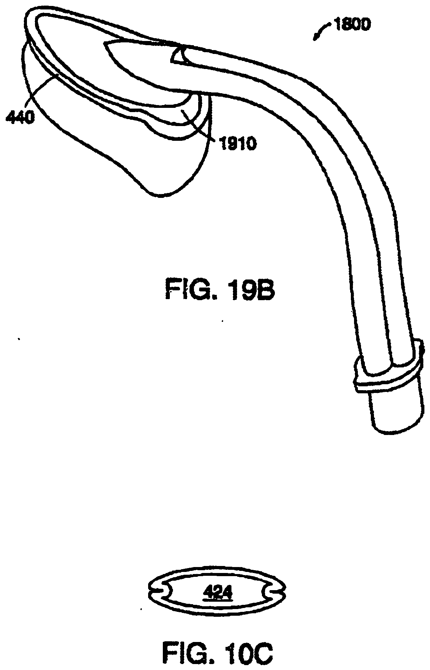

As indicated in Figure 10B, bending of the tube 410 (about an axis extending in the left-to-right direction) caused by inserting the LMA through the patient's anatomical airway generates compressive forces in the directions indicated by arrows 260. The longitudinal folds 425 tend to prevent localized collapse of the internal passage 424 as a result of bending the tube. If the tube 410 is subjected to compressive forces in the direction of arrows 260 sufficiently large to deform the tube, the tube may deform to the shape illustrated in Figure 10C. As shown, the deformation of the tube in the region of the longitudinal folds 425 may be likened to the movement of an accordion or concertina. The size of the internal passage 424 does decrease as the tube compresses from the profile shown in Figure 10B to the profile shown in Figure 10C. However, once the airway tube has reached the configuration shown in Figure 10C, the longitudinal folds 425 resist additional decreases in the size of the passage 424, even in response to additional compression of the tube. So, airway tube 410 advantageously (1) reduces the size of the inter-dental gap required for accommodating the tube; (2) provides a large airway passage; (3) decreases the likelihood that the tube will form kinks when the LMA is inserted into a patient; (4) decreases the likelihood that the tube will form kinks in response to bending of the patient's neck over the likely range of head movement; and (5) fits well within the patient's anatomical airway.

Another advantage of the longitudinal folds 425 is that they provide a convenient groove 425-g for locating the inflation tube 490. Figure 12 shows a perspective view of an LMA 400 constructed according to the invention in which the inflation tube 490 has been glued into the groove 425-g that extends along the right side of the airway tube.

Another important feature of the airway tube 410 is the degree of curvature through which the central portion 418 extends. As discussed in U.S. Patent Application Serial No. 08/901,055, there is an optimum degree of curvature for the airway tube of an LMA that will allow the patient to remain in a "neutral position" while the LMA is in the fully inserted configuration. The neutral position is a position in which the patient is lying on their back and in which the patient's head is positioned, for example with a pillow, so that the geometric relation of the head to the rest of the body is the same as when the patient is standing uptight and looking forward. The LMA disclosed in the '055 application used a rigid airway tube, and as discussed in that application, for rigid airway tubes the optimum degree of curvature is between 125 and 135 degrees. This degree of curvature permits the patient to remain in the neutral position while the LMA is being inserted and after the LMA has been placed in the fully inserted configuration.

For convenience of exposition, the shape assumed by airway tube 410 when the tube is not any external forces shall be referred to as the "preformed configuration". As will be discussed below, since the airway tube 410 is somewhat flexible, it can deviate from the preformed configuration when the LMA is in use. Figure 9E shows the integral tube and backplate section 416 in its preformed configuration. As shown, the airway tube 410 is preferably manufactured so that when it is not subjected to any external forces, the central portion 418 follows a circular curve about an axis C (the axis C extending in the left-to-right direction and being perpendicular to the plane of the page in Figure 9E) from a proximal limit of curvature 426 to a distal limit of curvature 427. In one preferred embodiment, the angle theta between two rays extending from the axis C to the mid distal limits 426, 427 for the preformed configuration is 105 degrees plus or minus ten degrees. More preferably, the angle theta for the preformed 105 degrees plus or minus five Even more preferably, the angle theta is substantially equal to 105 degrees. In one preferred embodiment of an adult female size, the distance, or radius, R₁, between the axis C and the inner surface 410-i of airway tube 410 for the preformed configuration is substantially equal to forty millimeters plus or minus about three and the distance, or radius, R₂, between the axis C and the outer surface 410-o of airway tube 410 for the preformed configuration is substantially equal to fifty millimeters plus or minus about three millimeters.

The preferred degree of curvature for the preformed configuration of LMA 400 is different than for the rigid tube LMA disclosed in the above-referenced '055 application. This difference in curvature facilitates insertion of LMA 400. When an LMA is inserted into a patient, proper insertion begins by placing the mask portion into the patient's mouth so that the pharyngeal side of the mask is in contact with the patient's hard palate. At this point, in LMA's designed according to the '055 application, the curve in the rigid airway tube forces the proximal end of the airway tube to be pushed against the patient's chest, Positioning the end of the tube against the patient's chest makes inserting the LMA somewhat more difficult than if the proximal end could be positioned at a location that was spaced apart from the patient's body. However, the requirements of a rigid airway tube (which facilitates later insertion of an endotracheal tube) and allowing the patient to remain in a neutral position before, during, and after insertion, necessitates positioning the airway tube's proximal end against the patient's chest at the beginning of insertion.

Like the LMA of the '055 application, LMA 400 allows the patient to remain in a neutral position before, during, and after insertion. However, unlike the LMA of the '055 application, the proximal end of the airway tube of LMA 400 need not be positioned against the patient's body at any time during insertion. If the airway tube 410 of LMA 400 were rigid and were formed with the above-discussed preformed configuration, then the patient could not remain in a neutral position while the LMA was in the fully inserted configuration. Rather, the patient's head would have to be tilted backwards to allow the airway tube to fit into the patient's anatomical airway. However, since the airway tube 410 is not rigid, the tube can flex, or bend, slightly away from the preformed configuration as it is being inserted thereby allowing the tube to fit into fit into the anatomical airway of a patient that is in the neutral position. The curve of the preformed configuration of the central portion 418 of the airway tube preferably does not deviate far from the anatomical curve of 125 to 135 degrees so that the tube need not bend much to fit into the anatomical airway. However, the curve of the preformed configuration of the central portion 418 Preferably deviates somewhat from the anatomical curve of 125 to 135 degrees so as to eliminate the need for pressing the tube's proximal end against the patient's chest during insertion.

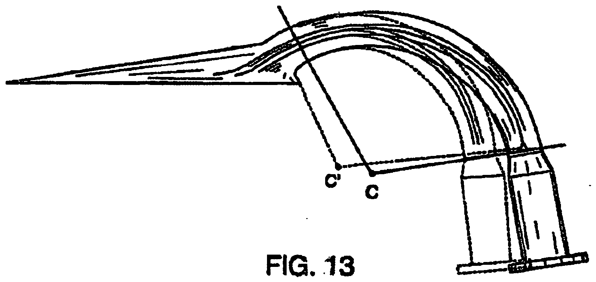

Figure 13 shows in solid lines a side view of integral tube and backplate section 416 in the preformed configuration. Figure 13 also shows in dotted lines the shape that integral tube and backplate section 416 assumes after the LMA 400 has been located in the fully inserted configuration within a patient that is resting in the neutral position. As shown, the airway tube 410 bends about an axis extending in the left-to-right direction when the LMA is inserted into a patient. When the LMA is inserted into a patient, the center or curvature, or axis about which the tube bends, shifts from C to C', and the angle through which the tube bends changes from the 105 degrees (plus or minus five or ten degrees) of the preformed configuration to the 125 to 135 degrees required to fit within the anatomical airway of a patient lying in the neutral position.

As discussed above, in one preferred embodiment, the integral airway tube and backplate section 416 is formed from polyvinyl chloride. This material is relatively stiff at room temperature but becomes much more flexible at body temperature. So, the airway tube is relatively stiff as the LMA 400 is being inserted into the patient. However, after the LMA 400 has been placed in the fully inserted configuration for a while (e.g., three to five minutes), the airway tube softens and becomes more pliable so that its shape easily accommodates to the shape of the patient's anatomical airway without placing undue force against the anatomical structures that define the anatomical airway. Also, since the material is relatively stiff at room temperature, the airway tube is generally stiff enough to act as an insertion tool. That is, LMA 400 may be entirely controlled during insertion simply by manipulating the portions of the airway tube 410 that extend outside of the patient's mouth. This eliminates the need for inserting a finger into the patient's mouth while inserting the LMA and further eliminates the need for additional insertion tools.

Another important advantage of LMA 400 relates to the quality of the seal provided with the laryngeal inlet. As shown in Figure 4A, there is a relatively large empty space S behind the mask portion 430. The empty space behind mask portion 430 is substantially larger than that provided by prior art LMAs and, as will be discussed below, advantageously allows LMA 400 to provide improved seals.

As shown in Figure 4A, the space S is defined by the distance T9 between the laryngeal side of the proximal end of the inflated cuff and the airway tube 410 as measured in the laryngeal-to-pharyngeal direction. A preferred value for the distance T9, when the airway tube is in the preformed configuration, is 32 millimeters plus or minus 3 millimeters. More preferably, the distance T9, when the airway tube is in the preformed configuration, is 32 millimeters plus or minus 2 millimeters. Even more preferably, the distance T9, when the airway tube is in the preformed configuration, is substantially equal to 32 millimeters.

When LMA 400 is in the fully inserted configuration, the posterior portion of the patient's tongue rests in the space S. As will be discussed below, enlarging the spare S in which the tongue rests improves the quality of the seal between the proximal end of the inflated cuff and the patient's laryngeal inlet.

Figure 14 shows a view of an inflated cuff of an LMA, and the illustrated cuff has been divided into three different regions. When the LMA is located in the fully inserted configuration, each region of the cuff contacts a different portion of the patient's anatomy. Region 1, at the cuffs proximal end, fits into the patient's valleculac (i.e., the space behind the lower part of the tongue). Region 2, which is disposed between the cuffs proximal and distal ends, contacts the patient's pyriform fossae, which are symmetrically disposed on either side of the patient's glottic opening. Region 3, which is disposed at the cuff's the distal end, contacts the patient's cricoid cartilage. Accordingly, when the LMA is inserted into a patient, a seal that extends continuously around the patient's glottic opening is formed by contact between the inflated cuff and the patient's valleculac, pyriform fossae, and cricoid cartilage.

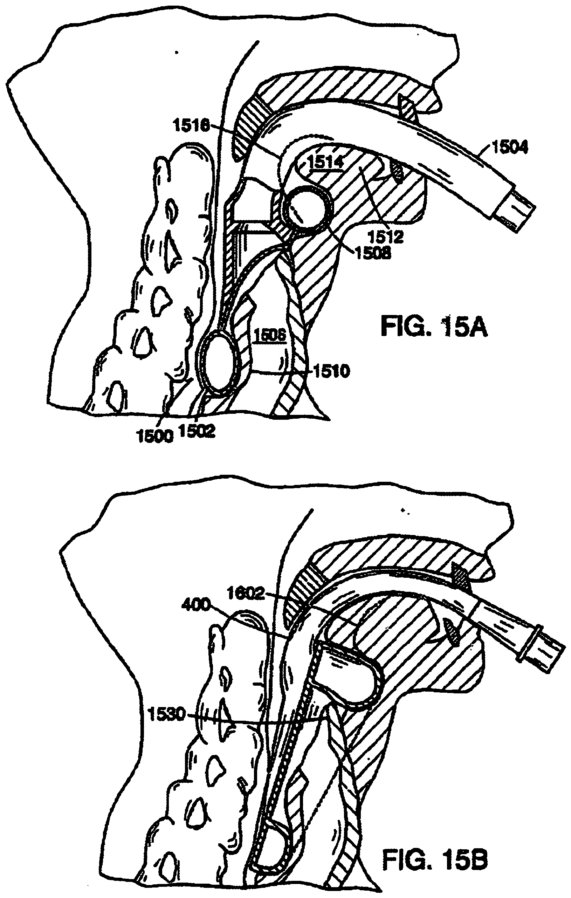

Figure 15A shows a prior art LMA 1500 that has been placed in the fully inserted configuration. As shown, the inflated cuff 1502 has formes a seal around the patient's glottic opening thereby coupling the passage of the airway tube 1504 to the patient's trachea 1506. The laryngeal side of the proximal portion of the cuff fits into the patient's valleculae 1508, and the laryngeal side of the distal portion of the cuff contacts the patient's cricoid cartilage 1510. The patient's tongue 1512 is disposed generally along the inner, or anterior, side of the airway tube between the patient's teeth and the proximal end of the inflated cuff. The posterior portion 1514 of the patient's tongue 1512 is disposed in the space S (between the proximal end of the inflated cuff and the inner, or anterior, of the side of the airway tube). The dashed line 1516 illustrates the contour the tongue 1512 would follow if the LMA 1500 were not inserted into the patient. As shown, insertion of the LMA displaces the tongue 1512 in the pharyngeal-to-laryngeal direction away from the natural position indicated by dashed line 1516. Pushing the tongue in this direction also pushes or levers portions of the larynx in the pharyngeal-to-laryngeal direction and thereby tends to prevent the cuff from fitting tightly around the larynx. This weakens the seal provided by the LMA by decreasing pressure between the cuff and anatomical structures such as the pyriform fossae.

Figure 15B shows LMA 400 in the fully inserted configuration. The dashed line 1602 represents the contour assumed by the tongue when prior art LMA 1500 is in the fully inserted configuration. As shown, the enlarged empty space S provided by LMA 400 allows the tongue to assume a more natural position than prior art LMA 1500. In particular, the enlarged empty space S of LMA 400 allows the tongue to be displaced in the laryngeal-to-pharyngeal direction from where the tongue would be if LMA 1500 were in the fully inserted configuration. Allowing the tongue to assume a more natural position also allows other anatomical structures to assume a more natural position (i.e., to be displaced in the laryngeal-to-pharyngeal direction from where they would be if LMA 1500 were in the fully inserted configuration) and thereby improves the seal provided by LMA 400.

As is well known, portions of the larynx (e.g., the ariepiglottic folds) can extend into the bowl shaped space bounded by the inflated cuff when an LMA is in the fully inserted configuration. Figure 15B suggests this by showing structures 1530 extending into the bowl-shaped volume defined by the cuff and backplate of LMA 400. Enlarging the space S also has the beneficial effect of increasing the size of the bowl-shaped volume defined by LMA 400 (i.e., increasing the empty space that is bounded by the backplate portion and the inflated cuff of LMA 400). This also improves the quality of the seal provided by LMA 400 by allowing the larynx to extend further into the bowl-shaped volume than was possible with prior art LMAs. Allowing the larynx to extend further into this space allows the larynx to assume a more natural position (i.e., a position similar to the position the larynx would occupy if the LMA were not inserted) and improves the seal provided by the LMA.

Several features of LMA 400 cooperate to provide the enlarged empty space S. First, as shown in Figure 5A, the thickness T5 of the proximal portion of the mask portion is substantially thicker than the thickness T4 of the distal portion of the mask portion. Another feature that cooperates to define the enlarged empty space S is the angle between the central portion 418 and the backplate portion 419 of the airway tube. As shown in Figure 4A, at the junction of the central portion 418 and the backplate portion 419, the central portion 418 extends at an angle alpha with respect to the plate 440. In one preferred embodiment, the angle alpha is equal to ten degrees plus or minus two degrees. More preferably, the angle alpha is equal to ten degrees plus or minus one degree. Even more preferably, the angle alpha is substantially equal ten degrees. This angle provides additional clearance between the proximal end of the plate and the inner side of the airway tube as measured in the laryngeal-to-phayneal direction. Yet another feature that contributes to defining the empty space is an absence of an inflation tube in the space. In most prior art LMAs, as shown for example in Figure 3, the inflation tube extends from the proximal end of the cuff in the distal-to-proximal direction into the space. However, in LMA 400, as shown for example in Figure 12, the inflation tube does not extend from the proximal end of the cuff and instead extends form the pharyngeal side of the plate to one of the notches 425 without entering the space S.

As discussed above, and as illustrated in Figures 5A-5C and 15B, one feature that helps define the enlarged empty space S is the increased thickness of the proximal end of the inflated cuff. When LMA 400 is in the fully inserted configuration, the inflatable cuff is preferably inflated to a pressure of about 60 cm H₂O. The pressure in the cuff tends to increase during surgical procedures because commonly used anesthesia gasses (e.g., nitrous oxide) tend to diffuse through the semi-permeable cuff wall. One advantage of forming mask portion 430 out of PVC is that a cuff formed of this material can hold the profile illustrated in Figures 5A-5C and 15B when the intra-cuff pressure rises due to this diffusion. In contrast, if the cuff were formed from a more elastic material, such as the silicone material used to form most prior art LMA cuffs, the cuff would not tend to hold this profile and would instead deform, or "balloon out", when intra-cuff pressure rises due to this diffusion.

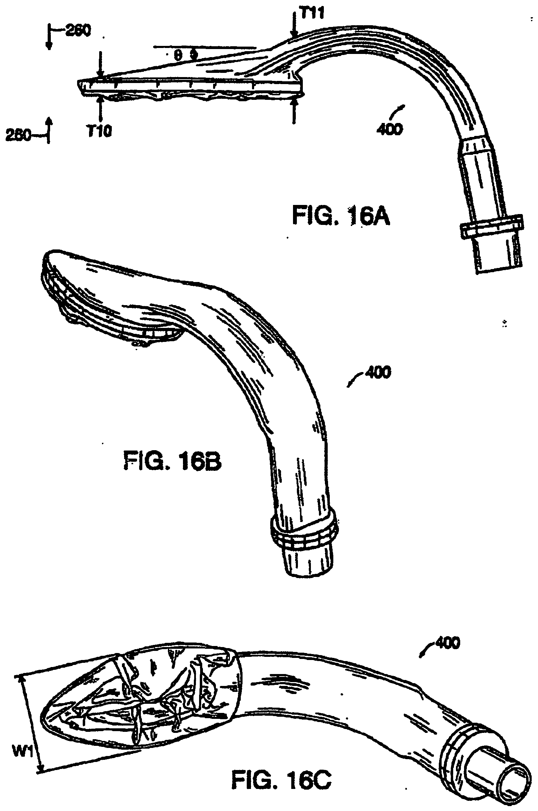

Yet another advantage of LMA 400 relates to the ease with which it can be inserted into a patient. Figure 16A shows a side view of LMA 400 when the cuff 460 is deflated. Figures 16B and 16C show perspective views of LMA 400 when the cuff 460 is deflated. The thickness T3 (as shown in Figure 6) of the cuff is sufficiently thin, that when the cuff 460 is deflated, the profile of the distal portion of the LMA is almost entirely determined by the plate 440 of the mask portion and the backplate portion 419 of the airway tube. As shown in Figure 16A, the thickness T10 of the distal end, as measured in the laryngeal-to-pharyngeal direction, is virtually entirely determined by the thickness of the plate 440. The thickness of the deflated LMA, as measured in the laryngeal-to-pha-yngeal direction, gradually increases with increases in the distal-to-proximal direction until the thickest point, at the proximal end of the mask portion, is reached which has a thickness T11, as measured in the laryngeal-to-pharyngeal direction. The rate of increase in thickness is determined by the angle theta between the plate 440 and the pharyngeal side of backplate portion 418. In preferred embodiments, the angle theta is about eleven degrees and the thickness T10 is about two millimeters (i.e., the deflated cuff adds virtually no thickness beyond the thickness of the plate T2). The thickness T11 is preferably about seventeen millimeters plus or minus two millimeters. More preferably, the thickness T11 is about seventeen millimeters plur, or minus one millimeter. Even more preferably, the thickness T11 is substantially equal to seventeen millimeters. The thickness T11, which is the thickest part of deflated LMA 400 as measured in the laryngeal-to-pharyngeal direction, is relatively thin as compared with prior art LMAs, which are usually about twenty-six millmeters thick in comparable sizes.

Figure 16C illustrates the size of the deflated LMA 400 as measured in the left-to-right direction. The width of the distal tip of the LMA is relatively narrow and the width of the LMA gradually increases with increases in the distal-to-proximal direction. The width of the widest part of the deflated LMA, as measured in the left-to-right direction W1 is equal to the width of the widest part of the plate (as shown in Figure 5E).

The overall profile of deflated LMA 400, as measured in the laryngeal-to-pharyngeal direction, as well as the left-to-right direction, is small as compared with prior art deflated LMAs. Having such a small profile greatly increases the ease with which deflated LMA 400 may be inserted into a patient. In particular, the thin profile, as measured in the laryngeal-to-pharyngeal direction, makes it very easy to push the deflated mask portion and backplate between a patient's upper and lower teeth and past the patient's threat. The thin profile also increases the likelihood that the deflated mask portion will fit between the pharyngeal wall and the epiglottis without disturbing or otherwise pushing on the epiglottis as the distal tip of the mask portion is being pushed past the epiglottis towards the esophageal sphincter.

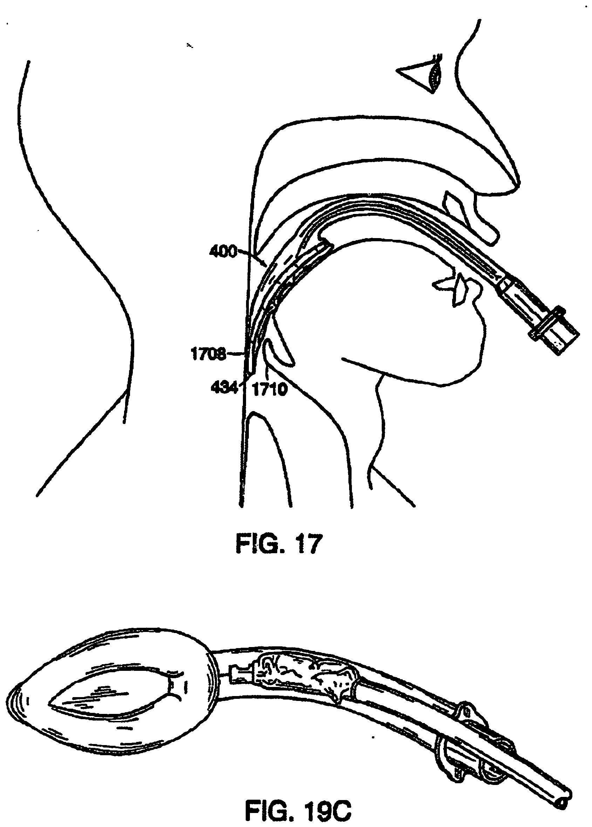

Figure 17 shows a deflated LMA 400 that has been partially inserted into a patient that is resting in the neutral position. As shown, the distal tip 434 of the deflated LMA has fit between the patient's pharyngeal wall 1078 and the epiglottis 1710. When an unconscious patient lies on their back, relaxation of the muscles tends to allow the back of the tongue and the epiglottis to drop down towards the pharyngeal wall, thereby reducing or minimizing the space between the epiglottis and the pharyngeal wall. Accordingly, the thinner the deflated LMA, the more likely it is that the LMA will fit into the space between the pharyngeal wall and the epiglottis without pushing on or otherwise moving the epiglottis. The slim profile of deflated LMA 400 accordingly facilitates proper insertion of the LMA.

One problem with prior art LMAs is that they are often inserted improperly. As discussed above, the LMA is a "forgiving" device and tends to establish an airway even when the device is improperly inserted. However, ideally, the LMA should be inserted properly so that the epiglottis is not disturbed and so that the distal tip of the LMA is disposed adjacent the esophageal sphincter. One problem that contributes to the difficulty of inserting prior an LMAs relates to the profile assumed by the deflated cuff. In prior art LMAs, the deflated cuff forms a "structural component" of the LMA in that (1) a significant portion of the profile of a deflated prior art LMA is determined by the cuff and (2) the shape of the deflated cuff significantly affects the path taken by the LMA through the body as it is inserted into a patient. Accordingly, proper insertion of a prior art LMA generally requires properly forming, or shaping, the cuff as it is deflated. U.S. Patent No. 5,711,293 discloses an example of a prior art forming tool for forming an LMA into an ideal shape for insertion as the cuff is being deflated.

In LMA 400, the deflated cuff contributes only insignificantly to the profile of the deflated LMA. Rather, the profile of the deflated device is determined almost entirely by the plate 440 of mask portion 430 and the backplate portion 419 of airway tube 410. As shown in Figure 16A-C, these components define a slim profile that facilitates proper insertion of the LMA.

Another advantage of LMA 400 relates to the profile of the device when deflated as compared with the profile of the device when inflated. As discussed above, when LMA 400 is deflated it presents a slim, thin, or small profile as compared with prior art LMAs. However, when LMA 400 is inflated, the cuff expands considerably and, as discussed above, this allows the LMA to provide an improved seal with the tissues surrounding the patient's glottic opening. The relatively large difference between the thickness (as measured in the laryngeal-to-pharyngeal direction) of the deflated device as compared with the thickness of the inflated device distinguishes LMA 400 from prior art LMAs. As discussed above, the thickest part of the deflated LMA, T11, is about seventeen millimeters. The thicken part of the inflated LMA, T5, is about 25.4 millimeters. Accordingly, the thickest part of the inflated LMA 400 is approximately 1.5 times larger than the thickest part of the deflated LMA 400. Although 1.5 is a preferred factor for distinguishing the thickest parts of the inflated and deflated LMA, it may be preferable for the thicken part of the inflated LMA to be 1.5, plus or minus 0.15, times larger than the thickest part of the deflated LMA (i.e., *T*5=(1.5±0.15)·*T*11).

As shown in Figure 17, any LMA will bend or flex as the LMA is being inserted into a patient. More specifically, as the distal tip of the LMA contacts the patient's palato-pharyngeal arch, the distal tip bends down towards the larynx (or bends about an axis that extends in the left to right direction). As the LMA is inserted further into the patient, the portion of the LMA that is proximal to the palato-pharyngeal arch will bend around the arch and portions of the LMA that have already passed by the palato-pharyngeal arch will straighten out. In this manner, the point of bending or flexing begins at the LMA's distal tip and moves backwards in the distal-to-proximal direction as the LMA continues to be inserted into the patient.

As shown for example in Figure 16B, the backplate portion 419 of LMA 400 is "spear shaped" or tapered in that its width decreases with increases in the pmximal-to-disud direction. The very narrow width of the bookplate's distal tip makes the LMA's distal tip relatively flexible so that the distal tip easily bends or flexes dow-nwards towards the larynx as the LMA 400 is inserted into the patient. As the LMA is inserted further, and the LMA's resistance to bending increases in a linear fashion due to the gradual widening of the "spear shaped" bookplate portion. This linear increase in resistance to bending about an axis that extends in the left-to-right direction is an advantageous feature of LMA 400. If the increase in resistance were not linear and instead increased suddenly or dramatically (in a non-linear fashion) at one or more points as the LMA was being inserted, the LMA would tend to kink, or form a localized fold, instead of bending smoothly around the palato-pharyngeal arch. Such a kink-like deformation would be more stimulating to the patient and increase the likelihood of malposition and/or trauma during insertion. Some prior an LMAs me capable of offering a substantially linear increase in resistance to bending as the LMA is inserted into a patient as long as the cuff has been properly deflated and formed into a proper configuration. However, since the cuff of these prior art LMAs forms a structural component of the LMA, they do not offer a linear increase in resistance to bending, and tend to form kinks while being inserted, when the cuff is deflated without proper use of a forming tool. One advantage of LMA 400 is that the LMA will provide the desired substantially linear increase in resistance to bending regardless of the manner in which the cuff is deflated. This is so because the deflated cuff does not contribute significantly to the structure of the LMA and the LMA's resistance to bending is virtually entirely determined by the geometry of the backplate portion 419.

Yet another advantage of LMA 400 relates to the size of the inflated cuff. As shown for example in in Figures 5A and 15A, the thickness T5, as measured in the pharyngeal-to-laryngeal direction, of the proximal end of the inflated cuff is relatively large as compared with prior art LMAs. The relatively large thickness T5 of the proximal end of the inflated cuff advantageously increases the separation between the epiglottis and the aperture 442 of plate 440 and thereby decreases the likelihood that the epiglottis can block the airway provided by the LMA 400. Prior an LMAs often included "bars" or or "slits" disposed in the mask portion to prevent the epiglottis from blocking the airway of the LMA. Such bars are disclosed for example in U.S. Patent No. 5,297,547 (see Figure 8 of the '547 patent). Although LMAs constructed according to the invention could include such "bars", LMA 400 advantageously eliminates the the need for such bars and accordingly may be manufactured less expensively.

Returning to Figure 17, as shown the distal tip of LMA 400 has passed through the gap between the epiglottis and the pharyngeal wall. Sometimes the distal tip of the LMA will catch on the epiglottis as the LMA is being inserted and will push the epiglottis into a "down folded" condition. In such a "down folded" condition, the epiglottis may block the trachea or the airway provided by an LMA. Another advantage of LMA 400 is that the cuff 460 can lift a down folded, or posterior lying, epiglottis forwards, or anteriorly, thereby keeping the airway clear. Figure 7B illustrates a preferred folded configuration for the deflated cuff. As shown, when the cuff 460 is deflated, the extra or loose material of the cuff may be folded towards the center of the mask portion so that the deflated cuff covers the entire, or nearly the entire, central aperture 442 of plate 440. If the cuff is folded into this position so that it covers the entire, or newly the entire, central aperture 442, then the cuff 460 will advantageously lift the epiglottis anteriorly and thereby open the airway as the cuff the is inflated.

One disadvantage of prior art re-usable LMAs is that after every sterilization, the cuff must be deflated and the LMA must be configured for insertion into a patient. Unfortunately, most physicians who use LMAs lack the skill or dedication required to pack the LMA into the optimal configuration for facilitating insertion. Another advantage of LMA 400 is that when it is used as a disposable device, the LMA may be packaged and sold in a configuration that is optimal for facilitating insertion of the device into a patient. As discussed above, LMA 400 is advantageous because (1) the deflated cuff only adds a small amount of thickness to the mask portion and (2) the deflated cuff may be configured for lifting a down folded or posterior lying epiglottis out of the way. Preferably, the LMA 400 is placed into this optimal configuration (i.e., with the cuff deflated and folded as discussed above in connection with Figures 7A and 7B) prior to sale and then packaged into a sterile bag or package (e.g., a sterile plastic bag). So, when a physician wishes to insert an LMA into a patient, the physician may simply remove an LMA from its sterile packaging and insert it into the patient without having to first deflate or reposition the cuff.

As discussed above, in some embodiments of LMA 400 an inflation tube 490 need not be provided. So, in embodiments that do not include inflation tubes, fabrication of the LMA is completed by attaching the airway tube to the partially inflated mask portion after the mask portion is removed from the mold. When mask portion 430 is formed by rotational molding, the cuff is partially inflated when the mask portion is removed from the mold. The amount of air that is napped in the cuff during fabrication is similar to the amount of air that is normally injected into the cuff via the inflation tube after the mask portion has been inserted into a patient to achieve the desired intra-cuff pressure of 60 cm H₂O. Accordingly, such a partially inflated cuff is capable of forming an effective seal around a patient's laryngeal inlet.

These masks have one principal disadvantage as compared with embodiments of LMA 400 that do include an inflation tube. The profile of the partially inflated cuff is thicker, as measured in the proximal-to-distal direction, than is achievable in LMA 400 when the cuff is fully deflated via the inflation tube, and this can make inserting the LMA more difficult. However, LMAs that do not include an inflation tube do have one principal advantage. Namely, can be easier and faster to use in emergency situations because the practitioner need not bother with deflating or inflating the cuff, and the airway is established as soon as the mask portion is inserted into the patient's pharynx. The thicker profile can complicate insertion of such an LMA. However, two factors make the insertion easier than might otherwise be the case. First, in unconscious patients, the muscles of the body become very relaxed which can make it easier to push a thick profile device through the upper and lower teeth and down the throat. Second, since the cuff is only partially inflated, and since the cuff is very thin and flexible, a very small amount of pressure applied to one portion of the cuff will squeeze, or shrink the size of that portion, and force air trapped in the cuff into other portions of the cuff thereby inflating or expanding those other portions. For example, the proximal end of the cuff will expand if the distal end is squeezed flat, and only a very small pressure is required to squeeze the distal end into a flat shape. As an LMA 400 with a partially inflated cuff is inserted into a patient, some parts of the cuff may expand while other parts are squeezed by anatomical structures. However, the ability to shrink in some places while expanding in others makes it relatively easy to push the partially inflated cuff into the patient's pharynx.

Accordingly, one method of making an LMA according to a preferred embodiment of the invention is to (1) produce mask portion 430 using the rotational molding process described above in connection with Figures 8A-8D; (2) remove mask portion 430 from the mold 800; and (3) attach an airway tube to the mask portion. The rotational molding process produces a partially inflated mask portion that is inflated to a suitable degree. Once the airway tube is attached to the mask portion, fabrication of the LMA is complete. An inflation tube need not be added. The completed LMA any be packaged for sale in a sterile bag. Such LMAs may be very useful for emergency situations, for example for use by emergency workers in ambulances or emergency wards.

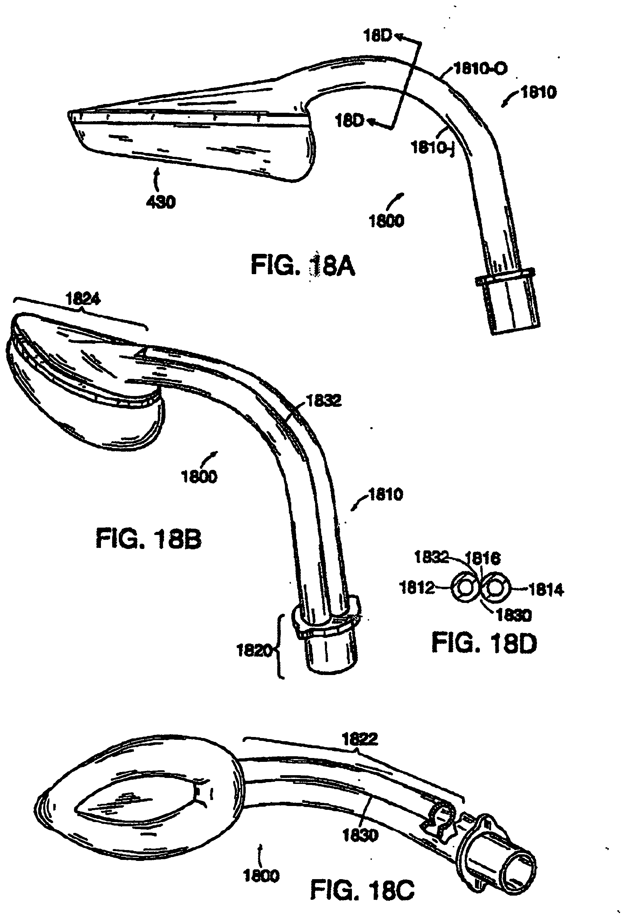

Figure 18A shows a side view of another embodiment of an LMA 1800 constructed according to a preferred embodiment of the invention. Figures 18B and 18C show two perspective views of LMA 1800. As shown, LMA 1800 is very similar to LMA 400. Both LMA 1800 and LMA 400 include identical mask portions 430. Also, the backplate of both LMAs 1800 and 400 are very similar. The principal difference betweem the two LMAs is in the airway tube.

The airway tube 1810 of LMA 1800 is a double barreled tube. Figure 18D shows a sectional view of airway tube 1810 taken in the direction indicated by line 18D-18D as shown in Figure 18A. Airway tube 1810 includes a left tube 1812 and a light tube 1814. The tubes are fixed, bonded, or extruded together at a central joint 1816 that extends from the proximal ends to the distal ends of the two tubes. Airway tube 1810 also defines an inner side 1810-i and an outer side 1810-o.

As with airway tube 410, tube 1810 has an overall or flattened cress section. Accordingly, tube 1810 (like tube 410), fits relatively well within the patient's anatomical airway and minimizes the intm-dental gap required to accommodate the tube. Also as with tube 410, airway tube 1810 includes a proximal portion 1820, a central portion 1822, and a backplate portion Bookplate portion 1824 is almost identical to backplate portion 419. The only principal difference between the two backplate portions is how they couple to their respective central portions of the airway tube.

As shown in Figure 18D, the junction of the two cylindrical tubes 1812 and 1814 at the joint 1816 forms two grooves, or recesses, 1830, 1832 in the airway tube. The groove 1830 extends along the inner side 1810-i of the airway tube and the groove 1832 extends along the outer side 1810-o of the tube. One advantage of tube 1810 is that the groove 1830 can serve as a guide for guiding subsequently inserted tubes, such as for example an endotracheal tube. That is, after LMA 1800 has been positioned in the fully inserted configuration, the groove 1830 can be used to guide a subsequently inserted device. Figure 19A shows a perspective view of an endotracheal tube being guided by groove 1830 as the endotracheal tube is inserted into the patient's body (not shown).

Embodiments of LMA 1800 that are used to guide a subsequently inserted endotracheal tube (or some other kind of tube), preferably define a "gap", or the and the at the "gap", or aperture, between the mask portion and the backplate portion at the proximal end of the mask portion. When the distal tip of the endotracheal tube reaches the mask portion's proximal end, continued insertion of the endotracheal tube will push the endotracheal tube's distal end through the gap between the mask portion and the backplate of the LMA and enable the endotracheal tube's distal end to proceed through the aperture 442 of the mask portion and into the patient's trachea.

Figure 19B shows an embodiment of LMA 1800 that defines such a gap 1910. Both LMA 400 and LMA 1800 are constructed by attaching or bonding the outer perimeter of the laryngeal side of the backplate portion of the airway tube to the pharyngeal side of the plate 440 of the mask portion 430. In the case of LMA 400, the entire outer perimeter of the backplate portion is so attached to the plate 440. However, in the case of LMA 1800, one portion of the outer perimeter of the backplate (at the backplate's proximal end) is not bonded to the plate 440 and the rest of the outer perimeter of the backplate is bonded to the plate 440. Since the proximal ends of the backplate and plate 440 are not bonded together, pressure on the plate 440 can push the plate 440 of the mask portion away from the backplate and create the gap 1910. In the absence of downward pressure on the plate 440, the portions of the backplate and plate 440 that are bonded together tend to hold the unbonded portions together as well. The effect is to create an LMA that has a "flap valve". Under normal conditions, the plate 440 and backplate of LMA 1800 remain in contact as in the case of LMA 400. when LMA 1800 is in the fully inserted configuration, pressure exerted by the patient's pharyngeal and laryngeal walls tends to push the plate 440 and bookplate towards one another, or together. However, in LMA 1800, pressure on the proximal end of the mask portion (generated for example by subsequent insertion of an endotracheal tube that is guided by groove 1830) can push the plate 440 away from the backplate to generate the gap 1910. Subsequently inserted endotracheal tubes can extend through gap 1910 and then through aperture 442 and into the patient's trachea.

Figure 20 shows a perspective view of an alternative embodiment of a mask portion 430' that may be used in LMA's. Mask portion 430' is similar to mask portion 430, however, the pharyngeal side of the plate 440' of mask portion 430' is not flat and instead defines a step, or 2010, that extends around the elliptical central aperture of the mask portion, It will be appreciated that the recess 2010 may be used to properly locate the bookplate portion of the airway tube when the backplate portion is fixed to the mask portion. Preferably, the laryngeal side of the backplate portion is bonded or fixed to the bottom of the recess 2010. When the backplate portion is fixed to the bottom of recess 2010, a small portion 2012 at the distal end of the plate 440' separates the distal tip of the backplate portion from the distal tip of the LMA. This may be advantageous because the airway tube is generally harder and stiffer than the mask portion. So, as the LMA is inserted into a patient, and the distal tip contacts anatomical structures within the patient's natural airway, the contact is between the patient and the relatively soft mask portion rather than between the patient and the harder backplate portion. Mask portion 430' thereby advantageously provides a simple mechanism for properly locating the backplate portion when the LMA is being assembled and also protests the patient from potential traumatic contact with the relatively hard distal tip of the backplate portion as the LMA is being inserted. It will be appreciated that mask portion 430' may be used in place of mask portion 430 in LMA 400, LMA 1800, or any other L MAs.

As discussed above in connection with Figures 10B and 10C, the longitudinal folds in the airway tube permit the tube to compress somewhat in a concertina or accordion like fashion. Another advantage of the longitudinal folds is that can permit the airway tube to expand in response to forces applied to the interior of the tube. This expansion can advantageously permit the airway tube to accommodate a subsequently inserted endotracheal tube and thereby allows LMA 400 to function as an intubating LMA. Figure 10D shows a side view of an embodiment of LMA 400 into which an endotracheal tube 1010 has been inserted. To reach the configuration illustrated in Figure 10D, the distal end 1012 of endotracheal tube 1010 was inserted into the proximal end of integral tube and backplate section 416 and advanced through the section 416 until the distal end 1012 emerged through the aperture in the mask portion 430 as shown. As the endotracheal tube 1010 advances through integral tube and backplate section 416, the longitudinal folds in the section 416 allow the section 416 to expand and thereby accommodate the endotracheal tube.

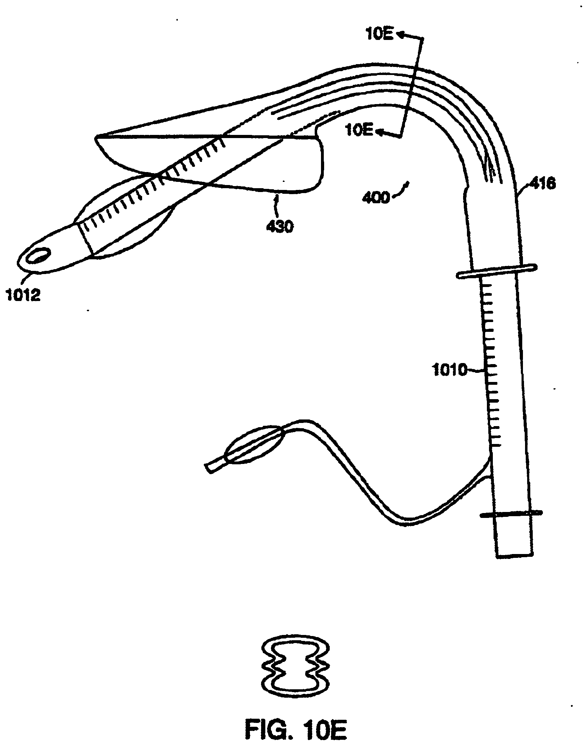

It will be appreciated that when LMA 400 is used as an intubating LMA, it may be desirable to use alternative embodiments of the airway tube 410 or the integral tube and backplate section 416. For example, the integral tube and backplate section 416 shown in Figure 10D includes two longitudinal folds that extend down the left and right sides of the tube rather than the single fold provided in the section 416 illustrated in Figure 10B and 10C. Figure 10E shows a cross section of the section 416 taken in the direction of line 10E-10E as shown in Figure 10D. Figure 10E shows the two longitudinal folds that extend down the left and right sides of the integral tube and backplate section. Figure 10E shows the integral tube and backplate section in an expanded condition. That is, the longitudinal folds have expanded in a concertina like fashion to accommodate the subsequently inserted endotracheal tube. It will be appreciated that airway tubes constructed according to the invention may be provided with one, two, or more longitudinal folds that extend down the left and right sides of the tube.

In addition to including extra longitudinal folds, it will be appreciated that it may be advantageous for the airway tube, or integral tube and backplate section, of intubating LMAs constructed according to the invention to include a modified proximal end that is cylindrical or otherwise wide enough to accommodate insertion of an endotracheal tube as shown in Figure 10D.

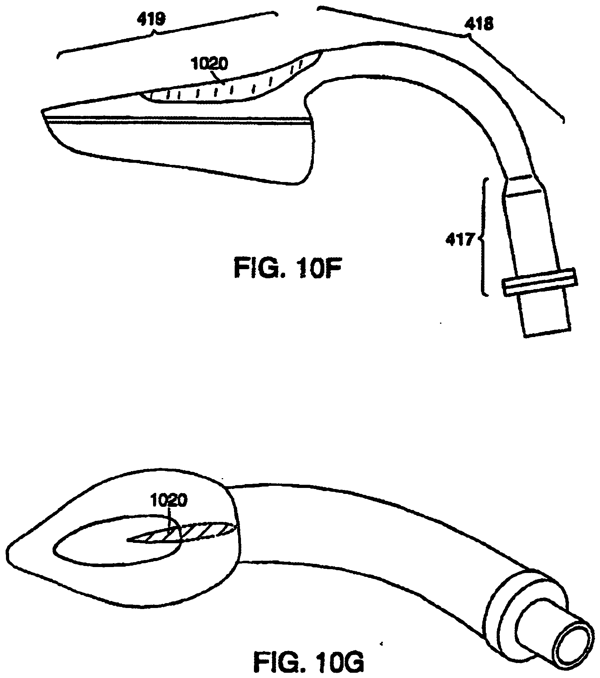

Figure 10F shows a side view or another embodiment of LMA 400 constructed according to a preferred embodiment of the invention; and Figure 10G shows a perspective view of the embodiment shown in Figure 10F. In the illustrated embodiment, the airway tube includes a ridge 1020. Ridge 1020 extends in the proximal-to-distal direction from a point the middle of the backplate portion 419 to a point in the curved portion 418 that is to a junction of the backplate portion 419 and the curved portion 418. Ridge 1020 also extends from the outer side of the tube 410-o into the interior of the passage defined by the In this embodiment, the wells of the tube near the junction of the curved portion 418 and the backplate portion 419 are also preferably weaker than the walls in other portions of the tube. For example, the tube wall can be made thinner in this region to weaken this portion of the tube.

The embodiment illustrated in Figures 10F and 10G facilitates rotating the patient's head while the LMA is in the fully inserted configuration. For example, the LMA may be placed in the fully inserted configuration while the patient is resting in the neutral position (i.e, the patient will be lying on their back and the patient's nose will be the part of the patient's head that is furthest from the ground). Once the LMA is so located, it may be desirable to rotate the patient's head. For example, if the patient's ear is being operated on, it may be desirable to rotate the patient's head approximately ninety degrees so that instead of the patient's nose, the patient's ear is now the part of the patient's head that is furthest from the ground. It will be appreciated that this exposes the ear and makes it easier to operate on the ear. Ideally, rotating the patient's head in this manner while the LMA is located in the fully inserted configuration (1) will not disturb the seal between the inflated cuff and the tissues surrounding the patient's glottic opening and (2) will not case a collapse of the internal passage provided by the airway tube. Weakening the walls of the airway tube new the junction of the backplate portion 419 and the curved portion 418 allows the distal part of the LMA (i.e., the mask portion and the backplate portion) to rotate with respect to the remainder of the airway tube without placing undue force on the inflated cuff, and this tends to preserve the seat between the cuff and the tissues surrounding the glottic opening when the patient"s head is so rotated. Ridge 1020 tends to prevent the internal passage provided by the airway tube from collapsing when the patient's head is so rotated and the airway tube is correspondingly twisted.

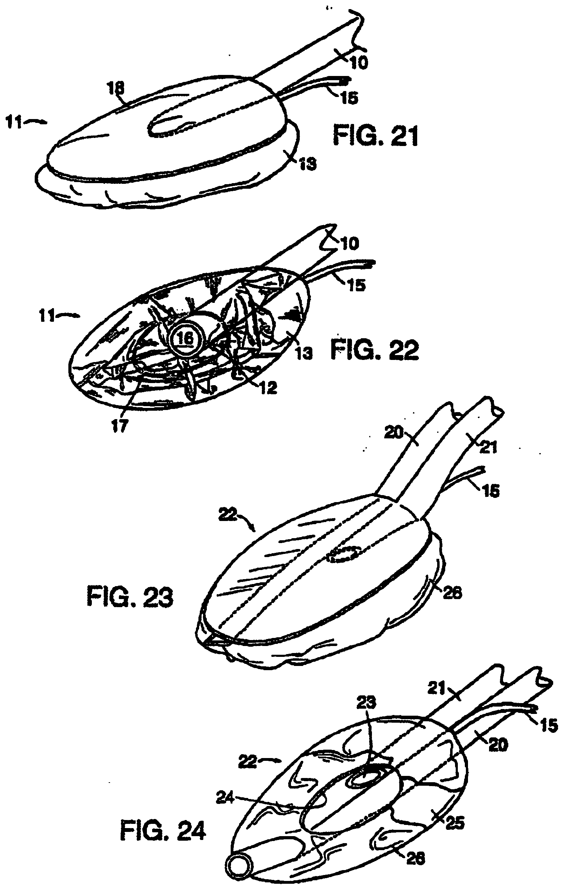

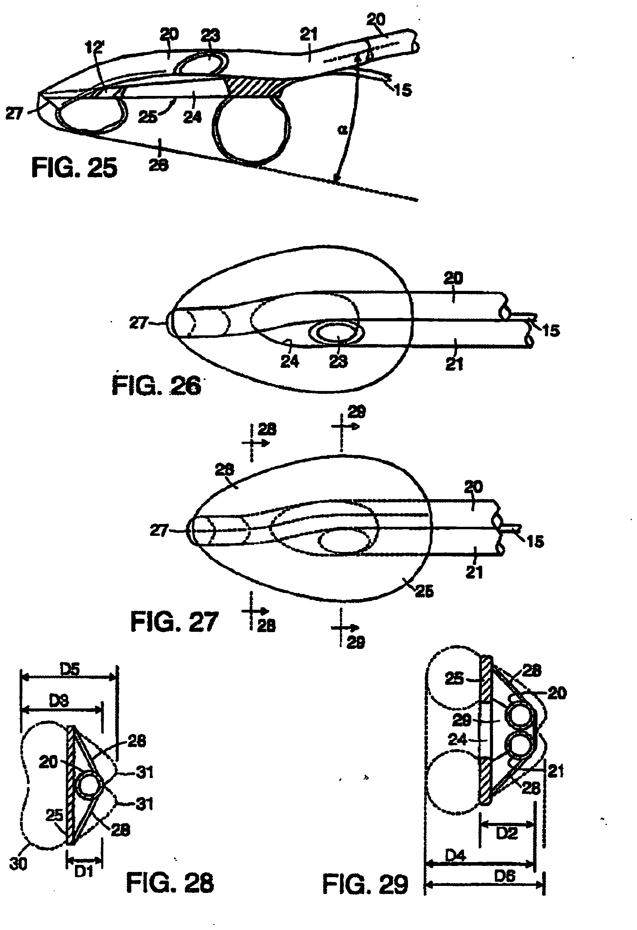

Figures 21 and 22 show another embodiment of an LMA constructed according to the invention. In this embodiment, an air inlet tube 10 will be understood to provide air (or other gas) service to a patient's lungs via mask structure 11 and the patient's trachea. As best seen in Figure 22, base structure of the mask 11 comprises a relatively stiffly pliant skeletal baste 12 of generally elliptical configuration, a portion of this base being viewable directly through a draftsman's break through a a collapsed thin-film inflatable envelope 13, which will be understood to be inflatable by external supply of inflation air via a flexible inflation line 15; line 15 will be understood to include a conventional two-way check valve (not shown) for purposes of holding an inflated condition of the envelope 13 (as in Figure 21) or for holding a deflated condition of the envelope (as in Figure 22). The envelope 13 is merely an inflatable portion of a single-part, integrally formed, total enclosure served by the inflation/deflation line 15, being the product of a so-called rotational-molding process, wherein a single plastic material in liquid state is caused to progressively build a thin layer or film of cured plastic material against and throughout the internal surface area of a given annular mould cavity, the gravitationally drained remained of the liquid-phase plastic being allowed to cure in situ as the relatively stiff skeletal annular member of the LMA, at the bottom of the mould. The cured product of such moulding not only provides the indicated skeletal-base function but also, between the inner and enter peripheries of the skeletal annulus provides the additional function of completing, as a skeletal annulus, the inflatable and peripherally yieldable enclosure of envelope provided by the moulded film. For the case of the described integrally formed component (12/13) when formed of suitable plastic such as polyvinylchloride, the thin film at 13 is typically of thickness in the order of 0.1 to 0.3 mm, while the skeletal base 12 may be typically 10 to 20 times the moulded thickness of the film 13. Such film will be understood to collapse and flatter or mat itself at random in response to deflation action via line 15. It is to be understood that while it is possible to form the skeletal base 12 as flat and of relatively uniform thickness, it is also possible to use the described moulding process to develop a skeletal-base thickness which varies as a function of longitudinal progression, as from a relatively thick proximal location (e.g., 2-3 mm thick) to a much reduced distal-end thickness (e.g., 1-mm), thereby according a desired distal-end bendability which can usefully serve the process of installing the LMA in the patient. Such a proximal-to-distal thickness variation is later indicated in Figures 25 (at 12') as a feature of the device of Figures 23 and 24.

To complete a description of the LMA device of Figures 21 and 22, the airway tube 10 is shown to be supported on and by its overlap with posterior surface of the proximal region of the annulus of skeletal base 12, the dismally open end 16 of the airway tube having preferably an angularly truncated configuration, which is open within the generally elliptical lumen 17 of the skeletal base 12. Finally, closure of the posterior side of the mask structure is effected by a tent-like roof 18 of flexible plastic sheet material, wherein the lapped distal portion of the airway tube is analogous to a ridge pole, so that the tent-like roof sheeting slopes away from its longitudinally central support by the distal end of the airway tube, to its peripherally scaled engagement to the rim of the skeletal base, as seen in Figure 21, it being understood that sheeting 18 is also suitably draped and sealed at its proximal-end closure around the airway tube 10.

Figures 23 and 24 are recognizable for their resemblance to Figures 21 and 22, except for the additional provision of a gastric-drainage tube 20, in side-by-side bonded relation to an airway tube 21, which may in all respects be as described for airway tube 10 of Figures 21 and 22, except for the fact that tubes 20/21 are symmetrically and oppositely offset from the longitudinal sagittal plane of the generally elliptical configuration of mask structure 22. This symmetrical relation is seen to continue until the distally open end 23 of the airway tube 21 is positioned to vent over the lumen 24 of the generally elliptical annular skeletal base 25 of the mask structure. As with the LMA of Figures 21 and 22, the base skeletal member 25 be a product of a rotational moulding operation wherein a thin-film inflatable/deflatable annular envelope 26 is integrally formed therewith, with provision for selective inflation/deflation action via a flexible line 15, as also in Figures 21 and 22.

For gastric-drainage purposes, and as better seen in Figures 25 to 29, the drainage tube 20 is seen in Figure 26 to undergo a mild zig-zag course change, from lateral offset adjacency to airway tube 21 to its distal-end alignment of symmetry with respect to the sagittal plane of the mask. Within the distal half of the skeletal base 25, and the distal end of drainage tube 20 passes through the base 25 and projects its angularly truncated open end 27 slightly beyond the distal end of base 25.

As previously noted, the longitudinal progression of reducing thickness of skeletal base 25 in the distal direction enables a more pliant action to be inherently imparted to the distal half of the mask. Figure 25 also illustrates that the inflated sectional area of the inflated thin-film envelope 26 is similarly and progressively decreased in the distal direction, so that tubes 20, 21 may be oriented at proximal departure from the mask to incorporate a preferred angle α in the range 20° to 30°, at commencement of their proximal course over the tongue, for air (gas) and gastric servicing connections (not shown), as necessary outside the patient's mouth.

As with the LMA of Figures 21 and 22, the structure of Figures 23 and 24 may be completed with a tent-like closure 28 of the posterior side of the mark. Again, such closure is realized by pliant sheet material which in Figure 28 is seen to derive "ridge-pole" support from tube 20, centered on the distal-half of skeletal base 25. In Figure 29, the section shows the tent closure 28 to be supported over the adjacent tubes 20, 21 at passage over the lumen 24 of the mask, with the skirt of tent sheeting peripherally secured to skeletal base 25, it being again understood that at its proximal end, the tent sheeting is also conformed and sealed to both tubes 20, 21 to complete closure of the posterior side of the mask.

In Figure 28, a bulging profile in phantom outline 30 on the anterior side of the mask will be understood to suggest film-envelope inflation away from the anterior surface of skeletal base 25, and a further inflation profile 31 in phantom outline on the posterior surface of the mask will be understood to suggest an inflatable cuff 31 over the periphery of base 25, a provide cushioned reference of the mask to the back wall of the patient's pharynx. As shown, the back-cushion material is shown for its further connection to tent 28 along the sagittal-plane intercept with tent 28.

It is desired that for case of installation of the mask in a patient, that the deflated condition should offer a minimum thickness dimension, this will be clear from Figures 28 and 29 where the respective minimum dimensions D1, D2 are to be compared with maximum available inflation dimensions D3, D4 without the back cushion 31, and D5, D6 with the back cushion 31.

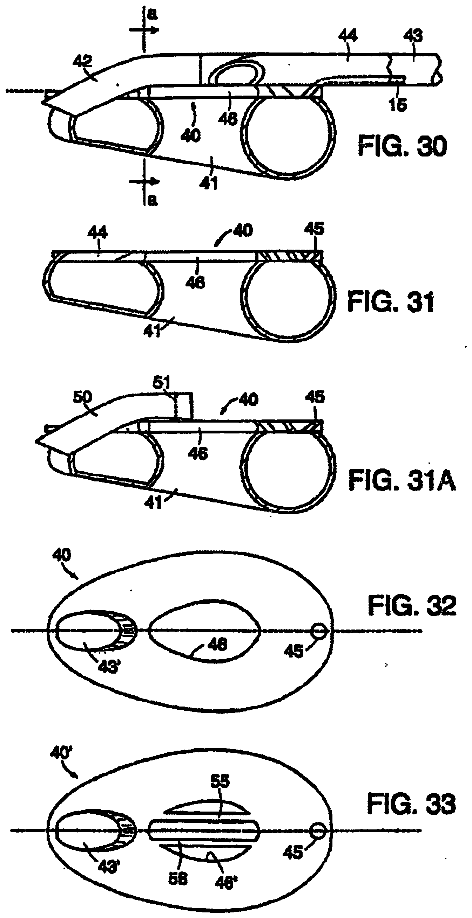

In the embodiment of Figures 30 to 32, the simplest difference to note is that the skeletal base 40 is flat and its integrally formed thin-film inflatable envelope portion 41 is otherwise as described for the inflatable film 26 of Figure 25. Also, the distal potion 42 of the drainage tube 43 is locally bent for straight but inclined passage through a similarly inclined orienting opening 44 in the distal-end region of base 40. At remaining overlap with the proximal-end region of base 40, the drainage tube 43 is laterally offset to the extent that it can symmetrically pair with airway tube 44, and both tubes 43, 44 can be banded to the supporting flat posterior surface of base 40. Tentlike sheet material described for closure of the posterior side of the mask can be as described for Figures 25 to 29, it being noted that at section a-a of Figure 30, the local section bean an almost identically similar appearance to that depicted in Figures 28 for the mask of Figure 27.

According to one technique of manufacture of the unitary base 40 with integrally moulded thin-film envelope portion 41, this single component is depicted in the longitudinal section of Figure 21 and in the plan view of Figure 22, it being understood that such passages as at 43' (for dminage-tube passage as at 43', for drainage-tube orientation), at 45 (for inflation-air access), and at 46 (for lumen definition) are the product of known core-pin and other mould-feature defining structures of the mould as an entirety. The preassembly of tubes 43 ,44 in side-by-side adjacency, together with the pre-bent and truncated open distal end of drainage tube 43 are later assembled for adhesively or otherwise scaled passage of the distal end of the drainage tube 43 and for film-pierced and peripherally sealed passage of the truncated distal end of tube 43 into the relationship depicted in Figures 30.

In an alternative mode of structural assembly, depicted in Figure 31A, a preformed and suitably beat distal-end fitting 50, for later assembly to the remaider of the drainage tube (not shown) is an insert part which in the process of rotation-moulding becomes the Figure 31A part to be later assembled to mask parts that become an LMA with the gastric-drainage feature. To this end, the preassembled drainage and airway tubes 43, 44 will be understood to terminate over the lumen 46 and that the distally projecting end of the dminage-tube portion (43) of this tube (43, 44) preassembly may be suitably fitted to the open proximal end of fitting 50, to establish continuity of the full drainage-tube function. Such continuity may be provided by known techniques of telescoping fit, as to the extent denoted by dotted line 51 in Figure 31A, or by a short sleeve of heat-shrink plastic material (not shown) which laps the abutting ends of equal diameter tubular ends, namely the proximal end of fitting 50 to the distal end of the two-tube preassembly (43, 44).

The plan view of skeletal base 40' of Figure 33 will be recognized as identical to that of Figure 32, except that two spaced elongate parallel bars 55, 56 Symmetrically staddle the longitudinal sagittal plane of the mask (not shown) into which this component can be integrated. The purpose saved by bars 55, 56 is to provide a measure of support for the drainage tube 43 as it passes over the lumen and as it alters course for distal-end symmetrical orientation with respect the sagittal plane.

## Claims

1. A laryngeal mask airway device (400), comprising a mask portion (430) and an airway tube (410), the airway tube extending from a proximal end (417) to a distal end, the distal end (416) of the airway tube being fixed to the mask portion, the mask portion including a backplate provided by the airway tube and being insertable through the mouth of a patient to an inserted location within the patient, the mask portion forming a seal around the patient's glottic opening when the mask portion is in the inserted location, the proximal end of the airway tube being disposed outside the patient when the mask portion is in the inserted location, **characterised in that** the mask portion has a durometer of fifty four plus or minus ten shore A, the airway tube has a durometer of seventy plus or minus fifteen Shore A and the device comprises polyvinyl chloride.

2. A method for forming a device as set in claim 1, the method comprising forming the airway tube by injection moulding.

## Patentansprüche

1. Kehlkopfmasken-Atemwegsvorrichtung (400), die einen Maskenabschnitt (430) und einen Atemwegstubus (410) umfasst, wobei sich der Atemwegstubus von einem proximalen Ende (417) bis zu einem distalen Ende erstreckt, wobei das distale Ende (416) des Atemwegstubus an dem Maskenabschnitt befestigt ist, wobei der Maskenabschnitt eine Rückplatte umfasst, die durch den Atemwegstubus vorgesehen ist und durch den Mund eines Patienten an eine Einfügstelle im Körper des Patienten eingefügt werden kann, wobei der Maskenabschnitt, wenn der sich an der Einfügstelle befindet, eine Versiegelung um die Glottisöffnung des Patienten bildet, wobei das proximale Ende des Atemwegstubus außerhalb des Patienten angeordnet ist, wenn sich der Maskenabschnitt in der Einfügstelle befindet, **dadurch gekennzeichnet, dass** der Maskenabschnitt einen Durometer von 54 ± 10 Shore-A aufweist, der Atemwegtubus einen Durometer von 70 ± 15 Shore-A und die Vorrichtung Polyvinylchlorid umfasst.

2. Verfahren zum Bilden einer Vorrichtung nach Anspruch 1, wobei das Verfahren das Formen des Atemwegstubus durch Spritzgießen umfasst.

## Revendications

1. Dispositif pour voie respiratoire à masque laryngé (400), comprenant une partie de masque (430) et un tube pour voie respiratoire (410), le tube pour voie respiratoire s'étendant d'une extrémité proximale (417) à une extrémité distale, l'extrémité distale (416) du tube pour voie respiratoire étant fixée à la partie de masque, la partie de masque comprenant une plaque arrière fournie par le tube de voie respiratoire et pouvant être insérée à travers la bouche d'un patient jusqu'à un emplacement inséré dans le patient, la partie de masque formant un joint autour de l'ouverture linguale du patient quand la partie de masque est à l'emplacement inséré, l'extrémité proximale du tube pour voie respiratoire étant disposée hors du patient quand la partie de masque est à l'emplacement inséré, **caractérisé en ce que** la partie de masque a un duromètre de cinquante-quatre plus ou moins dix Shore A, le tube pour voie respiratoire a un duromètre de soixante-dix plus ou moins quinze Shore A et le dispositif comprend du chlorure de polyvinyle.

2. Procédé de formation d'un dispositif selon la revendication 1, le procédé comprenant la formation du tube pour voie respiratoire par moulage par injection.
